# EUROPÄISCHE PATENTSCHRIFT

(11) **EP 3 359 094 B1**
(45) Veröffentlichungstag und Bekanntmachung des Hinweises auf die Patenterteilung: **01.01.2020**
(21) Anmeldenummer: 16778027.9
(22) Anmeldetag: 07.10.2016
(51) Int. Cl.: A61F 2/42, A61F 2/44, A61F 2/30

(54) **KÜNSTLICHES GELENKIMPLANTAT**
ARTIFICIAL JOINT IMPLANT
IMPLANT ARTICULAIRE ARTIFICIEL

(30) Priorität: 07.10.2015 DE 102015117129
(43) Veröffentlichungstag der Anmeldung: 15.08.2018
(73) Patentinhaber: Aesculap AG, 78532 Tuttlingen (DE)
(72) Erfinder: HAGEN, Thomas, 78532 Tuttlingen (DE); NONNENMANN, Martin, 78573 Wurmlingen (DE); MAAS, Allan, 78467 Konstanz (DE); GRUPP, Thomas, 78588 Denkingen (DE)
(74) Vertreter: Hoeger, Stellrecht & Partner Patentanwälte mbB
(86) Internationale Anmeldenummer: PCT/EP2016/073975
(87) Internationale Veröffentlichungsnummer: WO 2017/060413

(56) Entgegenhaltungen:
- EP-A1- 1 779 814
- WO-A1-2014/130224
- WO-A2-01/93786
- FR-A1- 2 761 878
- US-A1- 2005 113 919
- US-A1- 2006 200 239
- US-A1- 2010 152 856
- US-B1- 9 039 766

## Beschreibung

Die vorliegende Erfindung betrifft ein künstliches Gelenkimplantat mit einem ersten, an einem ersten Knochen oder Knochenteil anleg- oder festlegbaren Knochenanlageelement und einem zweiten, an einem zweiten Knochen oder Knochenteil anleg- oder festlegbaren Knochenanlageelement, wobei das erste und das zweite Knochenanlageelement relativ zueinander bewegbar angeordnet und gelenkig miteinander gekoppelt sind, wobei das Gelenkimplantat einstückig ausgebildet ist, wobei das Gelenkimplantat mindestens ein Festkörpergelenk umfasst, welches das erste und das zweite Knochenanlageelement gelenkig miteinander verbindet, wobei das mindestens eine Festkörpergelenk mindestens ein Federelement umfasst.

Künstliche Gelenkimplantate mit einem ersten, an einem ersten Knochen oder Knochenteil anleg- oder festlegbaren Knochenanlageelement und einem zweiten, an einem zweiten Knochen oder Knochenteil anleg- oder festlegbaren Knochenanlageelement, wobei das erste und das zweite Knochenanlageelement relativ zueinander bewegbar angeordnet und gelenkig miteinander gekoppelt sind, sind in vielfältiger Weise bekannt. Sie werden insbesondere eingesetzt, um geschädigte Hüft- und Kniegelenke zu ersetzen. Bekannt sind auch Bandscheibenprothesen in Form von Zwischenwirbelimplantaten, künstliche Schultergelenke sowie Sprunggelenksimplantate. Selbstverständlich ist diese Liste nicht abschließend.

Alle genannten künstlichen Gelenke ermöglichen es insbesondere, natürliche Gelenke, also bewegliche Verbindungen zwischen zwei oder mehreren Knochen, die beispielsweise in Folge eines Traumas oder aufgrund von Abnutzung geschädigt sind, zu ersetzen. Bei natürlichen Gelenken dient Gelenkknorpel, welcher an gelenknahen Knochenenden der das Gelenk ausbildenden Knochen ausgebildet ist, als Gleitfläche. Bei herkömmlichen Implantaten, insbesondere Gelenkimplantaten, die in der Orthopädie zum Einsatz kommen, sind die natürlichen Knorpel tragenden Knochenenden durch künstliche Komponenten ersetzt. Bei einem künstlichen Kniegelenk sind es beispielsweise eine Tibia- und eine Femurkomponente, wobei ein Gelenkspalt zwischen diesen beiden Komponenten mit einem künstlichen Medium ausgefüllt ist, insbesondere einem als Meniskuskomponente bekannten Gleitlagerelement. Um eine zwischen den Komponenten auftretende Reibung, die bei einer Relativbewegung derselben auftritt, zu reduzieren, werden insbesondere Teile der künstlichen Gelenke oder einzelne Komponenten der zusammenwirkenden Implantatteile beschichtet und/oder aus Kunststoff ausgeführt.

Eine unerwünschte Folge der auftretenden Reibung zwischen Komponenten des Gelenkimplantats sind mehr oder weniger viele Abriebpartikel, die in den Körper des Patienten gelangen und insbesondere Organe schädigen können. Das Auftreten von Abrieb wird zusätzlich verstärkt, wenn die einzelnen Implantatkomponenten relativ zueinander bei der Implantation nicht korrekt positioniert werden.

Die US 2010/0152856 A1 offenbart vollständig aus einem Metall ausgebildete dämpfende Zwischenwirbelimplantate. Aus der EP 1 779 814 A1 ist ein Implantat mit einstückigem Drehgelenk bekannt. Orthopädische Implantate sind in der WO 01/93786 A2 beschrieben. Eine implantierbare Vorrichtung zum Erhalten einer Beweglichkeit der Wirbelsäule ist in der US 2005/0113919 A1 offenbart. Eine Prothese, die dazu bestimmt ist, eine Beweglichkeit zwischen zwei Knochen wiederherzustellen, ist aus der FR 2 761 878 A1 bekannt. Wellfedern für Wirbelsäulenimplantate sind in der US 9,039,766 B1 beschrieben. In der WO 2014/130224 A1 sind Wirbelsäulenimplantate offenbart. Posteriore lumbale Zwischenwirbelstabilisatoren sind aus der US 2006/0200239 A1 bekannt.

Es ist daher eine Aufgabe der vorliegenden Erfindung, eine Körperverträglichkeit des künstlichen Gelenkimplantats der eingangs beschriebenen Art zu verbessern.

Diese Aufgabe wird bei einem Gelenkimplantat der eingangs beschriebenen Art erfindungsgemäß dadurch gelöst, dass das mindestens eine Federelement zwei oder mehr, äquidistant oder im Wesentlichen äquidistant voneinander angeordnete, wellenförmig gewundene Federglieder umfasst, welche zwei oder mehr Federglieder in Umfangsrichtung der platten- oder scheibenförmigen Knochenanlageelemente angeordnet sind, um Lagerelemente der beiden Knochenanlageelemente miteinander zu verbinden.

Das erfindungsgemäß vorgeschlagene künstliche Gelenkimplantat ermöglicht es insbesondere, vollständig auf Gleitpaarungen zu verzichten, sodass kein oder zumindest deutlich weniger Abrieb entsteht als bei herkömmlichen Gelenkimplantaten, die aneinander entlang gleitende Gelenkflächen aufweisen. Insgesamt ist das Gelenkimplantat aus einem einzigen Teil ausgebildet, sodass dessen innere Struktur eine Beweglichkeit zwischen dem ersten und zweiten Knochenanlageelement zulässt und definiert. Dadurch, dass das Gelenkimplantat einstückig ausgebildet ist, können insbesondere auch keine das Gelenkimplantat ausbildenden Teile vor oder während der Implantation verwechselt oder falsch eingesetzt oder in unerwünschter Weise relativ zueinander positioniert werden. Ein Operateur muss lediglich das Gelenkimplantat als Ganzes mit den verbliebenen Knochen oder Knochenteilen des Patienten verbinden, welche ursprünglich das zu ersetzende natürliche Gelenk des Patienten teilweise ausgebildet haben. Insbesondere kann das erfindungsgemäße Gelenkimplantat individuell an die Erfordernisse des zu ersetzenden Gelenks sowie die Physiologie des Patienten angepasst werden. Beispielsweise können das erste und/oder das zweite Knochenanlageelement patientenindividuelle Knochenanlageflächen aufweisen. Insgesamt können Gelenkimplantate so deutlich kostengünstiger hergestellt werden. Zudem lässt sich die Zahl der erforderlichen Einzelteile reduzieren und damit ein Aufwand bei der Herstellung, Lagerhaltung und beim Vertrieb verringern. Ein solches Gelenkimplantat ist insbesondere spiel-, verschleiß- und reibungsfrei, sodass ein Patient nicht mit Abriebpartikeln belastet wird. Außerdem kann so auf einfache Weise insbesondere auch ein Luxieren von Implantatkomponenten vermieden werden. Außerdem wird weniger Platz für das künstliche Gelenkimplantat benötigt, sodass ein Knochenabtrag im Vergleich zu herkömmlichen Gelenkimplantaten deutlich verringert ist. Es kann so also möglichst viel natürliche Knochensubstanz erhalten werden. Insbesondere auf aneinander anliegende und relativ zueinander bewegbare Oberflächen, wie sie bei herkömmlichen Gelenkimplantaten vorgesehen werden müssen, beispielsweise in Form von Gleitflächen, kann bei dem erfindungsgemäß vorgeschlagenen Gelenkimplantat verzichtet werden. So ist es insbesondere deutlich unempfindlicher gegenüber einwachsenden Weichteilen und eindringenden Körperflüssigkeiten. Dies nicht zuletzt deshalb, weil keine miteinander zusammenwirkenden, aneinander abgleitenden Gelenkflächen vorgesehen werden müssen. Optional kann das Gelenkimplantat aus zwei oder mehr Materialien oder Materialmischungen ausgebildet sein. Durch die einstückige Ausbildung des Gelenkimplantats kann insbesondere auf Klebe- und Fügeverbindungen sowie auf den Einsatz von Verbindungselementen wie Schrauben oder dergleichen zum Verbinden der Knochenanlageelemente verzichtet werden. Das Gelenkimplantat umfasst mindestens ein Festkörpergelenk umfasst, welches das erste und das zweite Knochenanlageelement gelenkig miteinander verbindet. Als Festkörpergelenk in diesem Sinne ist insbesondere ein Bereich, Abschnitt oder Teil des Gelenkimplantats zu verstehen, welcher eine Relativbewegung zwischen den ersten und zweiten Knochenanlageelementen ermöglicht, insbesondere durch Biegung oder Torsion. Die Knochenanlageelemente können insbesondere in Form starrer Körper ausgebildet sein, also beim einstückig ausgebildeten Gelenkimplantat Starrkörperbereiche bilden. Das mindestens eine Festkörpergelenk bildet also kein echtes Gelenk im Sinne eines kinematischen Paars, sondern beruht auf dem Prinzip der Elastostatik. Insbesondere können ein oder mehrere Bereiche verminderter Biege- und/oder Torsionssteifigkeit relativ zu zwei oder mehr angrenzenden Bereichen höherer Biege- und/oder Torsionssteifigkeit beim vorgeschlagenen Gelenkimplantat vorgesehen werden. Das mindestens eine Festkörpergelenk umfasst mindestens ein Federelement. Auf diese Weise ist es insbesondere möglich, eine Bewegung der Knochenanlageelemente aufeinander zu oder voneinander weg zu dämpfen. Zudem kann ein solches Festkörpergelenk auch auf einfache Weise tordiert werden. Durch die Ausgestaltung des Federelements können zudem zur Verformung des Festkörpergelenks erforderliche Kräfte vorgegeben werden. Beispielsweise kann das Federelement in Form einer Blattfeder ausgebildet sein. Das mindestens eine Federelement umfasst mindestens einen wellenförmig gewundenes Federglied. Ein solches lässt sich beispielsweise in Umfangsrichtung eines platten- oder scheibenförmigen Knochenanlageelements anordnen, um Lagerelemente der beiden Knochenanlageelemente miteinander zu verbinden. Es lässt sich auf einfache Weise komprimieren und dehnen. Insbesondere kann eine solche Verformung rein elastisch erfolgen. Das mindestens eine Federelement umfasst zwei oder mehr Federglieder. Insbesondere können diese äquidistant oder im Wesentlichen äquidistant voneinander angeordnet sein. Die Federglieder können spiralförmig oder auch wellenförmig gewunden sein. Insbesondere kann die Steifigkeit und Elastizität des Festkörpergelenks in Abhängigkeit der Zahl der eingesetzten Federglieder verändert werden.

Vorteilhaft ist es, wenn das mindestens eine Festkörpergelenk mindestens einen elastisch biegbaren Verbindungssteg umfasst oder durch einen solchen ausgebildet ist. Das mindestens eine Festkörpergelenk kann selbstverständlich auch zwei oder mehr elastisch biegbare Verbindungsstege umfassen. Durch die Zahl sowie die Elastizität der einzelnen Verbindungsstege kann eine Steifigkeit des mindestens einen Festkörpergelenks insgesamt eingestellt werden.

Um die gelenkige Verbindung des ersten und zweiten Knochenanlageelements miteinander in praktisch beliebiger Art und Weise einstellen zu können, ist es günstig, wenn das Gelenkimplantat zwei oder mehr Festkörpergelenke umfasst. Diese können insbesondere in unterschiedliche Richtungen wirkend die Knochenanlageelemente miteinander gelenkig verbinden.

Gemäß einer weiteren bevorzugten Ausführungsform der Erfindung kann vorgesehen sein, dass das mindestens eine Festkörpergelenk in Form eines Torsionselements ausgebildet ist. Eine Auslenkung der miteinander gelenkig gekoppelten Knochenanlageelemente kann so in Folge einer Torsion des mindestens einen Festkörpergelenks erfolgen. Das Torsionselement selbst kann wiederum aus mindestens einem elastisch verformbaren Verbindungssteg ausgebildet sein. Die Verbindungsstege können voneinander getrennt oder auch miteinander ganz oder abschnittsweise verbunden sein.

Um eine besonders hohe Stabilität des Gelenkimplantats zu erreichen, ist es günstig, wenn es mindestens zwei sich kreuzende Festkörpergelenke umfasst. Die mindestens zwei sich kreuzenden Festkörpergelenke können dabei miteinander in Kontakt stehen oder aber auch vollständig voneinander getrennt sein. Im letztgenannten Fall können sie insbesondere so ausgebildet sein, dass sie in Folge einer Auslenkung der miteinander gelenkig gekoppelten Knochenanlageelemente miteinander in Kontakt treten, beispielsweise um sich bei einer gewissen Auslenkung gegenseitig aneinander abzustützen. Dadurch kann insbesondere Gelenk auslenkungsabhängig steifer oder weniger steif eingestellt werden.

Vorteilhaft ist es, wenn sich die mindestens zwei Festkörpergelenke in einem Kreuzungsbereich kreuzen oder durchdringen oder wenn die mindestens zwei Festkörpergelenke in einem Kreuzungsbereich miteinander verbunden sind. Abhängig von der gewünschten Steifigkeit kann der Kreuzungsbereich der mindestens zwei Festkörpergelenke in der angegebenen Weise gestaltet werden. Durch das Verbinden der mindestens zwei Festkörpergelenke im Kreuzungsbereich kann eine Kopplung derselben erreicht und in definierter Weise eingestellt werden.

Um die Wechselwirkung und/oder das Zusammenwirken der mindestens zwei Festkörpergelenke im Kreuzungsbereich besonders gut definieren zu können, ist es günstig, wenn der Kreuzungsbereich in Form eines Kreuzungskörpers ausgebildet ist. Beispielsweise kann er in Form einer Hülse, insbesondere in Form einer zylindrischen Hülse, oder eines Quaders oder Würfels ausgebildet sein. Derartige Kreuzungskörper bieten insbesondere die Möglichkeit, Seitenflächen derselben mit Verbindungsstegen zu verbinden, deren anderes Ende mit einem der beiden Knochenanlageelemente verbunden ist.

Mehrere Festkörpergelenke lassen sich auf einfache Weise miteinander koppeln und in einem Kreuzungsbereich verbinden, wenn der Kreuzungskörper kugelförmig oder im Wesentlichen kugelförmig ausgebildet ist. So kann beispielsweise ein isotropes oder im Wesentlichen isotropes Gelenk ausgebildet werden.

Gemäß einer weiteren bevorzugten Ausführungsform der Erfindung kann vorgesehen sein, dass die mindestens zwei Festkörpergelenke einen unterschiedlichen Querschnitt aufweisen. Sie können alternativ auch einen identischen Querschnitt aufweisen. In beiden Fällen können sich die Festkörpergelenke kreuzen und/oder durchdringen. Insbesondere können die mindestens zwei Festkörpergelenke im Fall unterschiedlicher Querschnitte unterschiedliche Elastizitäten in unterschiedlichen Raumrichtungen aufweisen. Durch diese vorgeschlagenen Weiterbildungen lassen sich Beweglichkeiten, Torsions- und Biegesteifigkeiten der mindestens zwei Festkörpergelenke und damit auch des Gelenkimplantats insgesamt in gewünschter Weise einstellen.

Um die Implantation des Gelenkimplantats zu vereinfachen, ist es günstig, wenn das erste Knochenanlageelement eine ebene oder im Wesentlichen ebene Knochenanlagefläche aufweist und/oder wenn das zweite Knochenanlageelement eine ebene oder im Wesentlichen ebene Knochenanlagefläche aufweist. Ebene Knochenanlageflächen lassen sich auf einfache Weise mit ebenen Knochenflächen verbinden, beispielsweise mittels eines Verbindungsmittels wie zum Beispiel Knochenzement und/oder durch Einsatz von Knochenbefestigungselementen, insbesondere Knochenschrauben oder Nägel. Alternativ kann es auch günstig sein, wenn eine oder beide Knochenanlageflächen patientenindividuell geformt sind.

Das Ein- und/oder Anwachsen von Knochen an mindestens einer der beiden Knochenanlageflächen kann verbessert werden, wenn mindestens eine der beiden Knochenanlageflächen mit einer osteointegrativen Beschichtung versehen ist. Vorzugsweise sind beide Knochenanlageflächen mit einer osteointegrativen Beschichtung versehen.

Günstig ist es, wenn sich das mindestens eine Festkörpergelenk parallel oder im Wesentlichen parallel zu einer Knochenanlagefläche erstreckt. Insbesondere kann sich ein Verbindungssteg, welcher vom mindestens einen Festkörpergelenk umfasst ist, parallel oder im Wesentlichen parallel zu einer Knochenanlagefläche erstrecken. Das Festkörpergelenk kann beispielsweise als verbiegbares Element und/oder auch als Torsionselement ausgebildet sein, sodass eine Bewegung der beiden Knochenanlageelemente relativ zueinander praktisch in jede Raumrichtung möglich sein kann, insbesondere abhängig von der Ausrichtung und Orientierung des Festkörpergelenks.

Besonders gute Dämpfungseigenschaften für das Gelenkimplantat lassen sich erreichen, wenn das mindestens eine Festkörpergelenk das erste und das zweite Knochenanlageelement spiralförmig miteinander verbindet. Beispielsweise können zwei oder mehr Verbindungsstege vorgesehen sein, die gekrümmte, Ausschnitte aus einer Spirale definierende Verbindungselemente bilden, die die Knochenanlageelemente miteinander verbinden. Sie können sowohl eine Torsionsbewegung der Knochenanlageelemente relativ zueinander begrenzen als auch eine Bewegung derselben aufeinander zu oder voneinander weg.

Gemäß einer weiteren bevorzugten Ausführungsform der Erfindung kann vorgesehen sein, dass das erste Knochenanlageelement mindestens zwei erste Lagerelemente für freie Enden eines ersten Festkörpergelenks aufweist, dass das zweite Knochenanlageelement mindestens zwei zweite Lagerelemente für freie Enden eines zweiten Festkörpergelenks aufweist und dass sich das erste und das zweite Festkörpergelenk kreuzen oder durchdringen oder in einem Kreuzungsbereich miteinander verbunden sind. Durch diese Ausgestaltung lässt sich auf einfache Weise ein Gelenkimplantat ausbilden, dessen Knochenanlageelemente relativ zueinander bewegt werden können. Eine Verbindung zwischen den beiden Knochenanlageelementen kann beispielsweise direkt zwischen den Lagerelementen erfolgen oder indirekt indirekt über den Kreuzungsbereich.

Besonders einfach und kompakt ausbilden lässt sich das Gelenkimplantat, wenn die ersten und/oder zweiten Lagerelemente in Form von vom ersten oder zweiten Knochenanlageelement abstehenden Vorsprüngen ausgebildet sind. Beispielsweise können die Lagerelemente von den Knochenanlageelementen weg weisend in Richtung auf das jeweils andere Knochenanlageelement hin abstehend ausgebildet sein.

Günstig ist es, wenn die ersten und zweiten Lagerelemente jeweils einen Teil einer mindestens abschnittsweise umlaufenden Seitenwand des ersten Knochenanlageelements oder des zweiten Knochenanlageelements bilden und wenn die Seitenwände des ersten und zweiten Knochenanlageelements gemeinsam eine durch einen umlaufenden Schlitz getrennte Hülse definieren. Diese Ausgestaltung ermöglicht es insbesondere, mindestens ein Festkörpergelenk praktisch im Inneren des Gelenkimplantats, umgeben von den umlaufenden Seitenwänden sowie den Knochenanlageelementen, auszubilden. Dadurch ist das Festkörpergelenk gut geschützt. Eine ohnehin geringe Empfindlichkeit der Festkörpergelenkimplantate gegenüber einwachsenden Weichteilen und eindringenden Körperflüssigkeiten kann so noch weiter verringert werden. Zudem können insbesondere die durch den umlaufenden Schlitz getrennten Lagerelemente gegenseitig Anschläge definieren, um eine Bewegung der Knochenanlageelemente relativ zueinander zu begrenzen. Beispielsweise können die Lagerelemente Anschlagflächen aufweisen, an denen eine Anschlagfläche eines Lagerelements des jeweils anderen Knochenanlageelements bei einer extremen Auslenkung der Knochenanlageelemente relativ zueinander anschlagen.

Um eine Beweglichkeit der Knochenanlageelemente relativ zueinander in definierter Weise vorgeben zu können, ist es vorteilhaft, wenn ein Abstand des Schlitzes vom ersten Knochenanlageelement und/oder vom zweiten Knochenanlageelement in Abhängigkeit es Umfangswinkels variiert. So kann der Schlitz beispielsweise in bestimmten Umfangsbereichen näher beim einen und bestimmten anderen Umfangsbereichen näher beim anderen Knochenanlageelement angeordnet oder ausgebildet sein.

Vorzugsweise definieren den Schlitz begrenzende Randflächen der Seitenwände Anschlagflächen zur Begrenzung einer Relativbewegung der ersten und zweiten Knochenanlageelemente. Beispielsweise kann über eine Breite des Schlitzes, also über einen Abstand der den Schlitz begrenzenden Randflächen der Seitenwände, eine Auslenkung der Knochenanlageelemente aufeinander zu eingestellt werden. Zum Beispiel kann die Breite des Schlitzes in Abhängigkeit eines Umfangswinkels variieren, um so gezielt unterschiedlich starke Auslenkungen in unterschiedlichen Raumrichtungen zu gestatten.

Um eine zusätzliche Kontrolle einer Bewegung der Knochenanlageelemente relativ zueinander zu erreichen, ist es vorteilhaft, wenn jedes Lagerelement mit mindestens zwei weiteren Lagerelementen über Festkörpergelenke verbunden ist. Insbesondere können die Lagerelemente, die miteinander verbunden sind, am selben oder aber auch am anderen Knochenanlageelement angeordnet oder ausgebildet sein. Verbindungen der Lagerelemente miteinander können geradlinig, gekrümmt, beispielsweise in Form von Ausschnitten einer Zylinderoberfläche oder eines Schraubenelements, ausgebildet sein.

Vorteilhaft ist es ferner, wenn das erste und/oder das zweite Knochenanlageelement mindestens eine Führungsausnehmung für ein korrespondierendes Lagerelement des jeweils anderen Knochenanlageelements umfasst. Dies ermöglicht es insbesondere, eine Bewegung der Knochenanlageelemente relativ zueinander in definierter Weise zu führen. Insbesondere kann so auch eine Position der Knochenanlageelemente relativ zueinander bei einer bestimmten Auslenkung derselben aus einer Grundstellung des Gelenkimplantats vorgegeben werden.

Auf besonders einfache Weise ausbilden lässt sich das Gelenkimplantat, wenn die mindestens eine Führungsausnehmung in Form einer Nut ausgebildet ist. Beispielsweise kann ein Lagerelement in die Nut eintauchen und in dieser geführt werden.

Vorteilhafterweise ist die mindestens eine Führungsausnehmung in Richtung auf das Lagerelement des anderen Knochenanlageelements hin weisend konkav gekrümmt. So kann insbesondere eine rinnenartige Führung ausgebildet werden für zum Beispiel ein am anderen Knochenanlageelement angeordnetes oder ausgebildetes Lagerelement. Dieses kann insbesondere in Richtung auf die Führungsausnehmung hin konvex gekrümmt sein, um einen möglichst großflächigen Kontakt zwischen der Führungsausnehmung und dem Lagerelement zu erreichen.

Auf besonders einfache Weise herstellen lässt sich das Gelenkimplantat, wenn das mindestens eine Federelement in Form einer Schraubenfeder oder in Form Torsionsfeder ausgebildet ist. Durch die Gestalt derartiger Federelemente lassen sich Verformungskräfte für das mindestens eine Festkörpergelenk in gewünschter Weise und optional auch richtungsabhängig vorgeben.

Um die Steifigkeit beziehungsweise Elastizität des mindestens einen Festkörpergelenks in gewünschter Weise vorgeben zu können, ist es günstig, wenn das mindestens eine Federelement zwei oder mehr Federglieder umfasst. Zum Beispiel können diese in sich verschlungen sein. Derartige Federelemente können insbesondere ausgebildet sein ähnlich wie Gewindegänge mehrgängiger Schrauben.

Vorzugsweise umfasst das mindestens eine Festkörpergelenk einen Verbindungsstab. Dieser kann insbesondere dicker und weniger elastisch ausgebildet sein als ein Verbindungssteg. Insbesondere kann er dazu dienen, einen Mindestabstand zwischen den Knochenanlageelementen einzuhalten und vorzugeben sowie eine Querverschiebung oder Verschiebung der Knochenanlageelemente parallel zueinander minimieren.

Günstig ist es, wenn der Verbindungsstab quer vom ersten und/oder zweiten Knochenanlageelement abstehend ausgebildet ist. Insbesondere kann er senkrecht von einem oder beiden Knochenanlageelementen abstehen. Beispielsweise kann der Verbindungsstab zwei parallel zueinander angeordnete, scheibenförmige Knochenanlageelemente miteinander verbinden. Die Knochenanlageelemente können aber auch unter einem Winkel relativ zueinander geneigt sein, beispielsweise unter einem Winkel, der bei einer unverformten Wirbelsäule einem von zwei aufeinander zu weisenden Wirbelkörperendflächen definierten Neigungswinkel entspricht.

Um einen Grad sowie einen Ort der Verformung des Verbindungsstabs definiert vorgeben zu können, ist es günstig, wenn sich der Verbindungsstab ausgehend vom ersten und/oder zweiten Knochenanlageelement verjüngt. Beispielsweise kann der Verbindungsstab die Form eines Rotationshyperboloids aufweisen.

Gemäß einer weiteren bevorzugten Ausführungsform der Erfindung kann vorgesehen sein, dass das erste und/oder das zweite Knochenanlageelement in Form einer flachen Scheibe ausgebildet ist. Solche Knochenanlageelemente lassen sich auf einfache Weise herstellen, beispielsweise durch Spritzgießen oder durch 3D-Druck. Ferner ist es vorteilhaft, wenn die mindestens zwei Festkörpergelenke einen identischen Querschnitt aufweisen. Insbesondere kann es sich dabei um die sich kreuzenden Festkörpergelenke handeln. Die Querschnitte können optional auch unterschiedlich sein. Insbesondere kann dies der Fall sein, wenn unterschiedliche Elastizitäten in unterschiedlichen Raumrichtungen gewünscht werden.

Vorteilhafterweise ist ein Querschnitt des mindestens einen Festkörpergelenks kreuzförmig, sternförmig, vieleckig, rund oder oval. Grundsätzlich sind auch beliebige andere Querschnittsformen denkbar und möglich. Durch den Querschnitt kann insbesondere der Grad sowie die Richtung oder die Richtungen einer Verformung des mindestens einen Festkörpergelenks vorgegeben werden.

Um eine Bewegung der Knochenanlageelemente relativ zueinander dämpfen zu können, ist es vorteilhaft, wenn zwischen dem ersten und dem zweiten Knochenanlageelement ein Füllmaterial eingebracht ist. Beispielsweise kann das Füllmaterial dazu dienen, eine Bewegung der Knochenanlageelemente aufeinander zu zu dämpfen oder abzubremsen. Beispielsweise kann das Füllmaterial plastisch verformbar ausgebildet sein. Insbesondere ist es denkbar, in einer Hülle eingebrachte Kügelchen aus einem körperverträglichen Kunststoff zu verwenden.

Günstigerweise ist das Füllmaterial elastisch. Dies ermöglicht insbesondere eine elastische Verformung des Füllmaterials bei einer Bewegung der Knochenanlageelemente aufeinander zu und gegebenenfalls auch voneinander weg. Das Füllmaterial kann dann auch dazu dienen, das Gelenkimplantat im Sinne einer Rückstellreinrichtung - ähnlich wie in Form von Federelementen ausgebildete Festkörpergelenke - das Gelenkimplantat nach einer Auslenkung aus einer Grundstellung wieder in die Grundstellung automatisch zurück zu überführen.

Auf besonders einfache und kompakte Weise ausbilden lässt sich das Gelenkimplantat, wenn das Füllmaterial ein Kunststoff ist. Insbesondere kann es sich um Silikon oder oder einen anderen Kunststoff mit vergleichbaren elastischen Eigenschaften handeln.

Um eine Funktion des Gelenks dauerhaft zu gewährleisten und zudem ein Einwachsen von Weichteilen und Eindringen von Körperflüssigkeiten in das Gelenk oder Zwischenräume desselben zwischen den Knochenanlageelementen und/oder dem mindestens einen Festkörpergelenk zu vermeiden, umfasst das Gelenkimplantat vorzugsweise eine äußere Gelenkhülle oder Gelenkkapsel. Auf diese Weise lässt sich das Gelenkimplantat insgesamt einhüllen.

Vorteilhafterweise verschließt die Gelenkhülle oder Gelenkkapsel den umlaufenden Schlitz. Das Eindringen von Körperflüssigkeiten oder das Einwachsen von Körpergewebe können so im Wesentlichen oder ganz vermieden werden.

Ferner kann vorgesehen sein, dass die Hülle oder die Kapsel und/oder die Gelenkhülle oder die Gelenkkapsel mit einem Fluid gefüllt sind. Insbesondere kann es sich bei dem Fluid um ein Gas oder um eine körperverträgliche Flüssigkeit handeln. Beispielsweise kann das Fluid Wasser oder eine isotonische Kochsalzlösung sein.

Um Abstoßungsreaktionen des Körpers zu vermeiden, ist es günstig, wenn das Gelenkimplantat aus einem implantierbaren Metall oder Kunststoff hergestellt ist.

Insbesondere ist es günstig, wenn das Metall ein Implantatstahl oder Titan ist oder enthält oder wenn der Kunststoff Polyetheretherketon (PEEK), Polyethylen (PE) und/oder Silikon ist oder enthält. Insbesondere kann der Implantatstahl ein Kobalt-Chrom-Stahl sein. Derartige Implantatmaterialien eignen sich hervorragend zur Ausbildung langlebiger Implantate.

Auf einfache Weise lässt sich das Gelenkimplantat herstellen, wenn es durch Spritzgießen und/oder 3D-Druck hergestellt ist. Insbesondere ermöglicht die Herstellung des Gelenkimplantats durch 3D-Druck die Ausbildung ineinander verschlungener und/oder einander durchdringender Elemente des Gelenkimplantats, insbesondere sich kreuzende oder sich umgebende oder durchdringende Festkörpergelenke.

Gemäß einer weiteren bevorzugten Ausführungsform der Erfindung kann vorgesehen sein, dass das Gelenkimplantat in Form eines künstlichen Sprunggelenks, eines Zwischenwirbelimplantats, eines Hüftgelenks, eines Kniegelenks, eines Schultergelenks, eines Finger- oder Zehengelenks ausgebildet ist. Insbesondere für Gelenke mit einem eingeschränkten Bewegungsspektrum wie insbesondere Sprunggelenke und Bandscheiben, lässt sich so auf einfache und kostengünstige Weise ein spiel-, verschleiß- und im Wesentlichen reibungsfreies Gelenkimplantat ausbilden, welches sowohl sehr leicht als auch nur geringe Geräusche verursachend ausgebildet werden kann. Festkörpergelenke verursachen in der Regel bei einer Verformung keine Geräusche. Zudem kann durch Herstellung des Gelenkimplantats mittels 3D-Druck auch ein patientenindividuelles Gelenkimplantat auf einfache Weise realisiert werden. Insbesondere können die Knochenanlageelemente patientenindividuelle Knochenanlageflächen aufweisen, die großflächig an einer natürlichen Knochenfläche, beispielsweise einer Seitenfläche eines Wirbelkörpers, welcher dann für die Implantation nicht mehr gesondert präpariert werden muss, oder einer präparierten Knochenfläche, beispielsweise einer distalseitig resezierten Tibia, angelegt werden können.

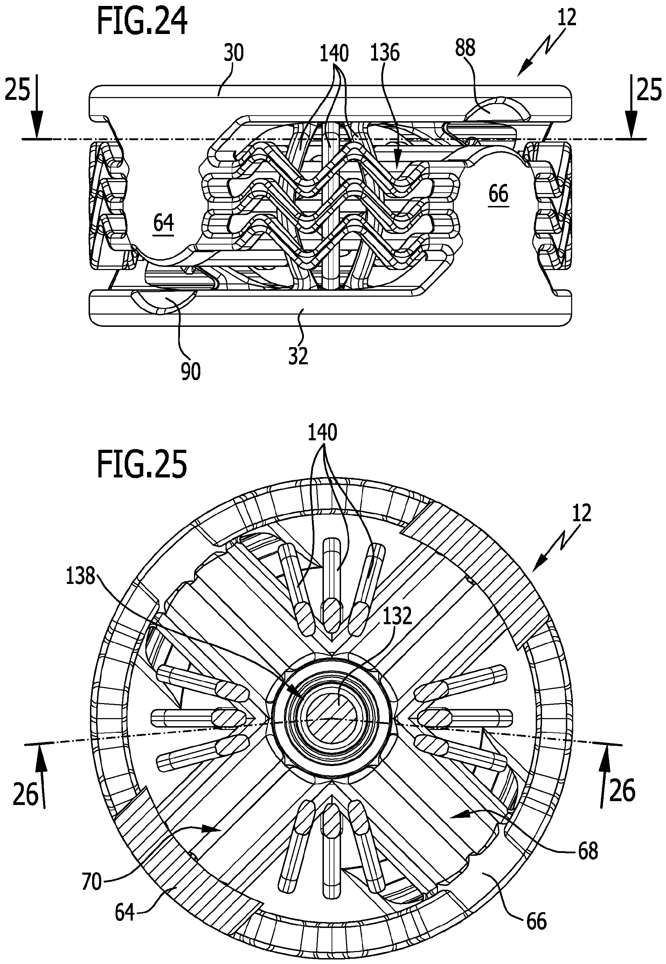

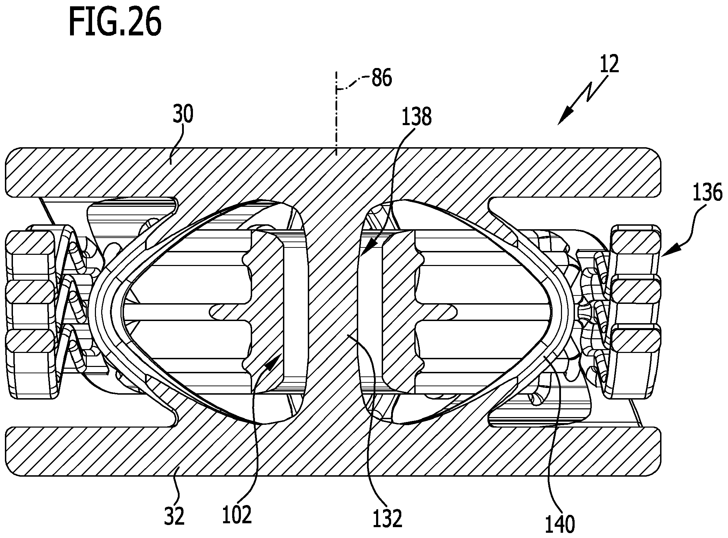

Die nachfolgende Beschreibung bevorzugter Ausführungsformen der Erfindung dient im Zusammenhang mit den Zeichnungen der näheren Erläuterung. Die beanspruchte Erfindung wird in den Fig. 19 bis 26 gezeigt. Es zeigen:
- Figur 1:: eine schematische perspektivische Ansicht eines Fußskeletts mit einer Sprunggelenksprothese in Form eines einstückig ausgebildeten Gelenkimplantats;
- Figur 2:: eine schematische perspektivische Ausschnittsansicht einer Wirbelsäule mit einem Zwischenwirbelimplantat in Form eines einstückig ausgebildeten Gelenkimplantats;
- Figur 3:: eine perspektivische, teilweise durchbrochene Gesamtansicht eines ersten Ausführungsbeispiels eines Gelenkimplantats;
- Figur 4:: eine Ansicht des Gelenkimplantats aus Figur 3 in Richtung des Pfeils A;
- Figur 5:: eine Ansicht des Gelenkimplantats aus Figur 3 in Richtung des Pfeils B;
- Figur 6:: eine Schnittansicht längs Linie 6-6 in Figur 4;
- Figur 7:: eine perspektivische, teilweise durchbrochene Gesamtansicht eines zweiten Ausführungsbeispiels eines Gelenkimplantats;
- Figur 8:: eine Ansicht des Gelenkimplantats aus Figur 7 in Richtung des Pfeils C;
- Figur 9:: eine Ansicht des Gelenkimplantats aus Figur 7 in Richtung des Pfeils D;
- Figur 10:: eine Schnittansicht längs Linie 10-10 in Figur 8;
- Figur 11:: eine perspektivische, teilweise durchbrochene Gesamtansicht eines dritten Ausführungsbeispiels eines Gelenkimplantats;
- Figur 12:: eine Seitenansicht der beiden sich kreuzenden Festkörpergelenke des Gelenkimplantats aus Figur 11;
- Figur 13:: eine Seitenansicht der Anordnung aus Figur 12 in Richtung des Pfeils E;
- Figur 14:: eine Seitenansicht eines vierten Ausführungsbeispiels von sich kreuzenden Festkörpergelenke mit kugelförmigem Kreuzungskörper im Kreuzungsbereich;
- Figur 15:: eine Ansicht auf die Anordnung aus Figur 14 in Richtung des Pfeils F;
- Figur 16:: eine perspektivische Ansicht eines fünften Ausführungsbeispiels sich kreuzendender Festkörpergelenke;
- Figur 17:: eine Seitenansicht der Anordnung aus Figur 16;
- Figur 18:: eine Ansicht der Anordnung aus Figur 17 in Richtung des Pfeils G;
- Figur 19:: eine perspektivische Gesamtansicht eines sechsten Ausführungsbeispiels eines einstückigen Gelenkimplantats;
- Figur 20:: eine teilweise durchbrochene Ansicht des Gelenkimplantats aus Figur 19 in Richtung des Pfeils H;
- Figur 21:: eine teilweise durchbrochene Ansicht des Gelenkimplantats aus Figur 19 in Richtung des Pfeils I;
- Figur 22:: eine Schnittansicht längs Linie 22-22 in Figur 23;
- Figur 23:: eine Schnittansicht längs Linie 23-23 in Figur 22;
- Figur 24:: eine Seitenansicht eines siebten Ausführungsbeispiels eines einstückigen Gelenkimplantats;
- Figur 25:: eine Schnittansicht längs Linie 25-25 in Figur 24;
- Figur 26:: eine Schnittansicht längs Linie 26-26 in Figur 25;
- Figur 27:: eine perspektivische Gesamtansicht eines achten Ausführungsbeispiels eines einstückigen Gelenkimplantats; und
- Figur 28:: eine Seitenansicht des Gelenkimplantats aus Figur 27.

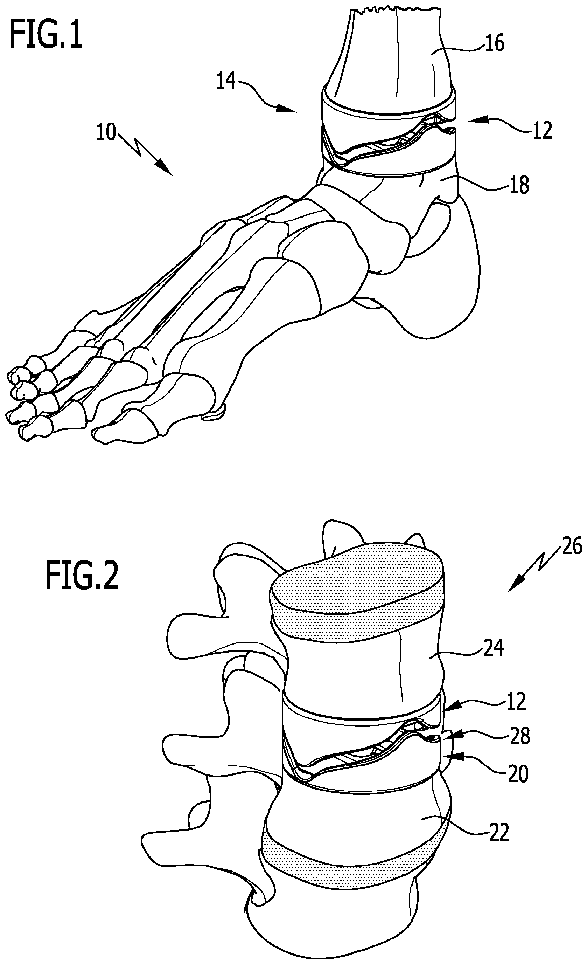

In Figur 1 ist schematisch ein Fußskelett 10 mit einem einstückig ausgebildeten Gelenkimplantat 12 in Form eines Sprunggelenkimplantats 14 dargestellt. Es dient zum Ersetzen eines geschädigten und vor einer Implantation des Gelenkimplantats 12 entfernten oberen Sprunggelenks.

Vorzugsweise wird das Gelenkimplantat 12 ohne Einsatz von Knochenzement, also zementfrei, an benachbarten Knochen 16 und 18 festgelegt. Hierfür können beispielsweise in nicht dargestellter Form vom Gelenkimplantat abstehende Vorsprünge vorgesehen sein, die in vor der Implantation des Gelenkimplantats 12 präparierte Aufnahmen an den Knochen 16 beziehungsweise 18 eingreifen können.

Ein weiteres Anwendungsgebiet für einstückige Gelenkimplantate 12 ist in Figur 2 dargestellt. Das Gelenkimplantat 12 dient hier als Zwischenwirbelimplantat 20 zwischen benachbarten Wirbelkörpern 22 und 24 einer Wirbelsäule 26. Mithin übernimmt das Gelenkimplantat 12 also die Funktion einer künstlichen Bandscheibe 28.

Das Gelenkimplantat 12 kann jedoch auch als Ersatz für beliebige andere geschädigte Gelenke eines menschlichen oder tierischen Patienten eingesetzt werden, beispielsweise und nicht abschließend somit auch als künstliche Hüft- oder Kniegelenke oder auch als Schultergelenke. Es versteht sich von selbst, dass geeignete Anpassungen für den jeweiligen Einsatzzweck am Gelenkimplantat 12 vorgenommen werden können beziehungsweise müssen.

Die nachfolgend beschriebenen Ausführungsbeispiele von Gelenkimplantaten 12 können insbesondere alle für die vorgenannten Einsatzzwecke genutzt werden.

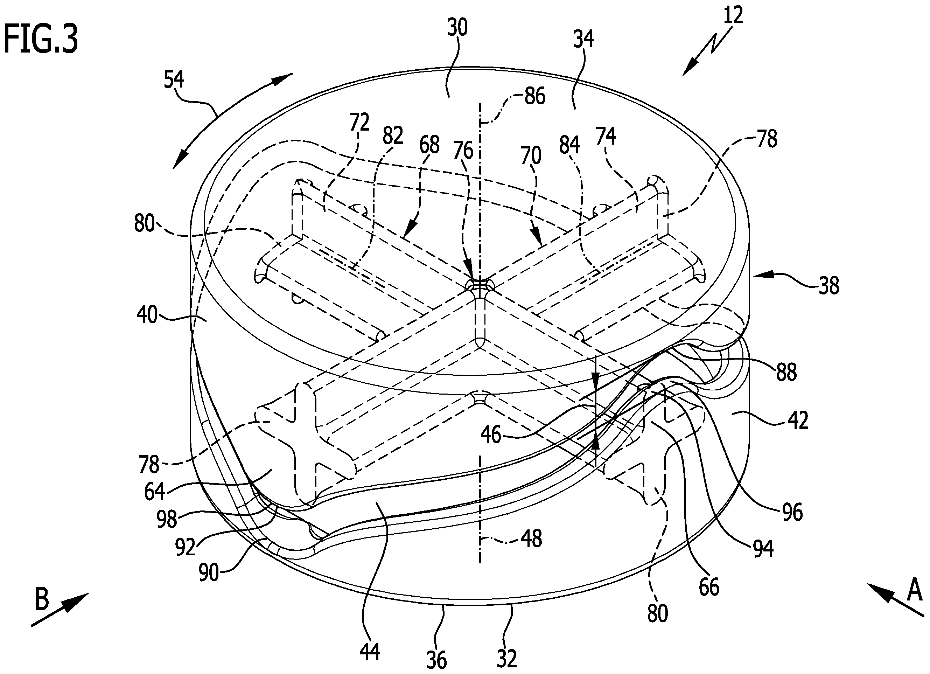

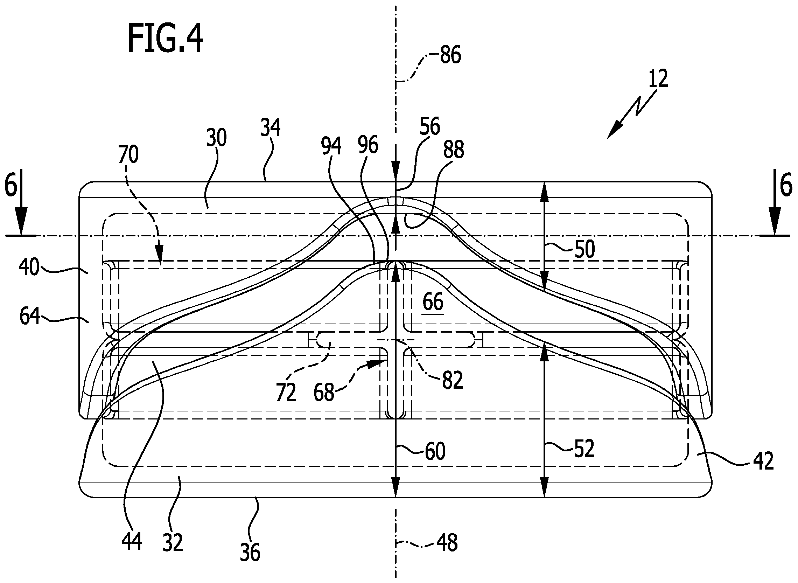

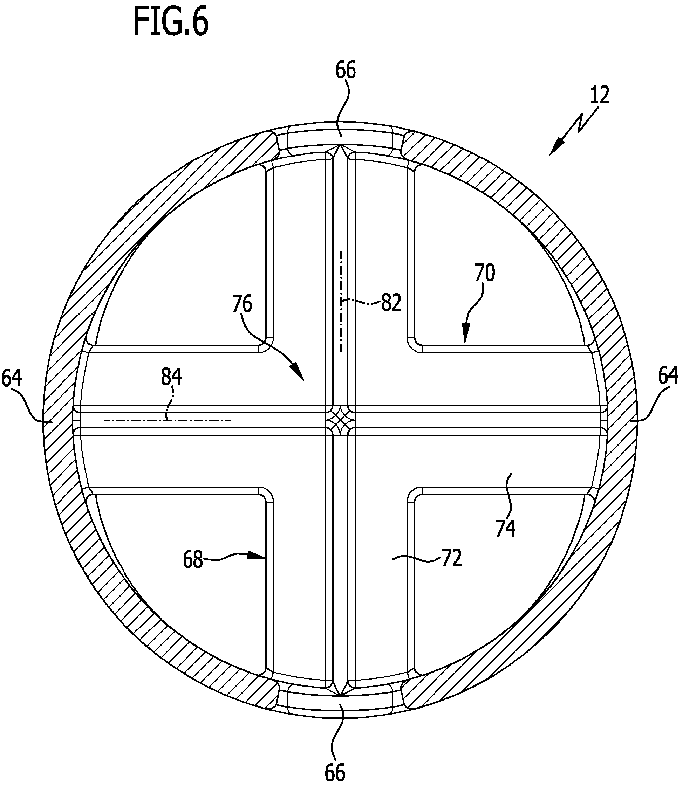

In den Figuren 3 bis 6 ist ein erstes Ausführungsbeispiel des Gelenkimplantats 12 beispielhaft dargestellt. Es umfasst zwei Knochenanlageelemente 30 und 32, die jeweils in Form einer Kreisscheibe ausgebildet sind.

Das Knochenanlageelement 30 definiert eine vom Gelenkimplantat weg weisende ebene Knochenanlagefläche 34. Das Knochenanlageelement 32 definiert eine ebenfalls vom Gelenkimplantat 12 und zudem in entgegengesetzter Richtung zur Knochenanlagefläche 32 weg weisende Knochenanlagefläche 36.

Die beiden in den Figuren eben dargestellten Knochenanlageflächen 34 und 36 können optional auch in Form patientenindividueller Knochenanlageflächen ausgebildet sein. In diesem Fall weisen sie eine Kontur auf, die einer Kontur einer äußeren Oberfläche beispielsweise der Knochen 16 oder 18 oder der Wirbelkörper 22 oder 24 entspricht, um eine eindeutige Implantationsposition für das Gelenkimplantat 12 im Körper des Patienten vorzugeben.

Eine äußere Kontur des Gelenkimplantats 12 wird definiert durch die Knochenanlageelemente 30 und 32 sowie eine hülsenförmige, die Knochenanlageelemente 30 und 32 außen begrenzende umlaufende Seitenwand 38. Die Seitenwand 38 wird gebildet durch einen ersten Seitenwandteil 40, der sich vom Knochenanlageelement 30 in Richtung auf das Knochenanlageelement 32 hin weisend erstreckt, sowie einen zweiten Seitenwandteil 42, der sich vom Knochenanlageelement 32 in Richtung auf das Knochenanlageelement 30 hin weisend erstreckt.

Die Seitenwand 38 ist umlaufend durch einen Schlitz 44 getrennt, so dass die beiden Seitenwandteile 40 und 42 durch den Schlitz 44 getrennt und voneinander beabstandet sind. Ein Abstand 46 der Seitenwandteile 40 und 42 voneinander entspricht einer Breite des Schlitzes 44 in einer Richtung 48, die senkrecht zu den Knochenanlageflächen 34 und 36 in einer Grundstellung des Gelenkimplantats 12 orientiert ist.

Eine Höhe 50 des ersten Seitenwandteils 40 sowie eine Höhe 52 des zweiten Seitenwandteils 42 bilden jeweils bezogen auf die Knochenanlageflächen 34 beziehungsweise 36 eine Funktion eines Umfangswinkels. Mit anderen Worten ändern sich die Höhen 50 und 52 in Umfangsrichtung. Bei dem in den Figuren 3 bis 6 beispielhaft dargestellten Ausführungsbeispiel des Gelenkimplantats 12 weist die Höhe 50 zwei absolute Minima 56 und zwei absolute Maxima 58 auf. Jeweils bei den Umfangswinkeln, bei denen die Höhe 50 ein Minimum 56 aufweist, weist die Höhe 52 ein absolutes Maximum 60 auf, wohingegen bei einem der Maxima 58 jeweils ein absolutes Minimum 62 der Höhe 52 ausgebildet ist.

Eine Breite des Schlitzes 44 ist unabhängig vom Umfangswinkel konstant beziehungsweise im Wesentlichen konstant. Die Funktionen der Höhe 50 und 52 sind im mathematischen Sinn stetig und stetig differenzierbar, weisen also kein Sprünge oder Knicke in Umfangsrichtung auf.

Durch die variablen Höhen 50 und 52 ändert sich also auch ein Abstand des Schlitzes von den beiden Knochenanlageelementen 30 und 32.

Die Minima 56 liegen einander diametral gegenüber, ebenso ist dies bei den Maxima 58 und 60 sowie den Minima 62 der Fall. Es wechseln sich also immer nach einem Umfangswinkel von 90° Minima 56 und Maxima 58 beziehungsweise Maxima 60 und Minima 62 miteinander ab.

Der erste Seitenwandteil 40 bildet im Bereich der Maxima 58 zwei Lagerelemente 64. Ebenso bildet der zweite Seitenwandteil 42 im Bereich der Maxima 60 zwei Lagerelemente 66.

Beim Gelenkimplantat 12 sind die Knochenanlageelemente 30 und 32 gelenkig miteinander verbunden. Zu diesem Zweck umfasst das Gelenkimplantat 12 zwei Festkörpergelenke 68 und 70, die, wie gut in den Figuren 3 bis 5 zu erkennen, jeweils einen kreuzförmigen Querschnitt aufweisen. Die Festkörpergelenke 68 und 70 sind somit in Form von im Querschnitt kreuzförmigen Verbindungsstegen 72 und 74 ausgebildet, die in einem Kreuzungsbereich 76 miteinander verbunden sind.

Freie Enden 78 und 80 der Verbindungsstege 72 beziehungsweise 74 sind an die Seitenwandteile 40 beziehungsweise 42 angeformt. Jeder Verbindungssteg 72 beziehungsweise 74 verbindet somit die Lagerelemente 64 beziehungsweise 66 eines Seitenwandteils 40 beziehungsweise 42 miteinander. Über den Kreuzungsbereich 76 sind dann Verbindungsstegabschnitte der Verbindungsstege 72 beziehungsweise 74 miteinander verbunden, so dass die Knochenanlageelemente 30 und 32 über die beiden Festkörpergelenke 68 und 70 dauerhaft gelenkig miteinander verbunden sind.

Die Festkörpergelenke 68 und 70 definieren jeweils eine Längsachse 82 beziehungsweise 84, die sich senkrecht schneiden und zu einer Mittelachse 86 senkrecht verlaufen. Die Mittelachse 86 verläuft parallel zur Richtung 48 und damit senkrecht zu den Knochenanlageflächen 34 und 36 in der Grundstellung des Gelenkimplantats 12.

Die Seitenwandteile 40 und 42 definieren im Bereich ihrer Minima 56 beziehungsweise 62 Führungsausnehmungen 88 beziehungsweise 90 für die jeweils gegenüberliegenden Lagerelemente 66 beziehungswiese 64.

Randflächen 92 beziehungswiese 94 der Seitenwandteile 40 beziehungswiese 42, welche aufeinander zu weisen, bilden somit auch Anschlagflächen 96 beziehungsweise 98, die eine Relativbewegung der Knochenanlageelemente 30 und 32 aufeinander zu begrenzen. Insbesondere können die Lagerelemente 64 und 66 in die korrespondierenden Führungsausnehmungen 90 beziehungsweise 88 eintauchen und so eine Relativbewegung der Knochenanlageelemente 30 und 32 definiert führen und begrenzen.

Die Festkörpergelenke 68 und 70 sind bei dem in den Figuren 3 bis 6 dargestellten Ausführungsbeispiel des Gelenkimplantats 12 im Wesentlichen als Torsionselemente ausgebildet, die eine Verwindung der Festkörpergelenke 68 und 70 um ihre Längsachsen 62 und 84 ermöglichen.

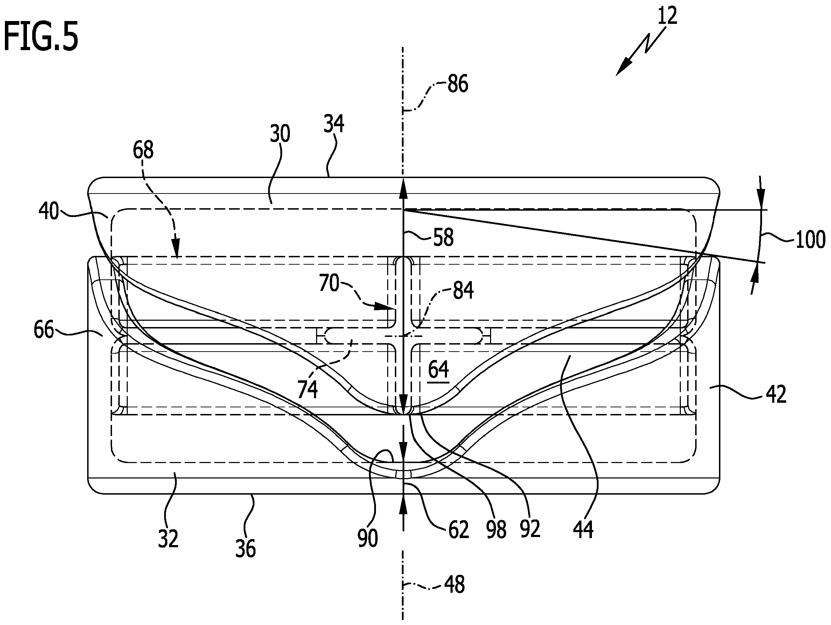

Der beschriebene Aufbau des Gelenkimplantats 12 ermöglicht somit eine definierte Verkippung der Knochenanlageflächen 34 und 36 relativ zueinander um einen maximalen Winkel 100, welcher sich ergibt, wie schematisch in Figur 5 dargestellt, aufgrund einer Beziehung eines Abstands der Randflächen 92 beziehungsweise 94 von der Mittelachse 86 und des Abstands 46, also einer Breite des Schlitzes 44.

In geringem Umfang ermöglicht das Gelenkimplantat 12 auch eine Verwindung, das heißt eine Verdrehung, der Knochenanlageelemente 30 und 32 relativ zueinander um die Mittelachse 86.

Das Gelenkimplantat 12 kann aus einem Metall oder einem Kunststoff gebildet sein. Beispielsweise können Kobalt-Chrom-Molybdän-Legierungen oder Titan zum Einsatz kommen. Ebenfalls denkbar ist die Herstellung des Gelenkimplantats 12 aus einem Kunststoff, beispielsweise Polyetheretherketon (PEEK).

Die Knochenanlageflächen 34 und 36 können optional mit einer osteointegrativen Beschichtung versehen sein, um das Anwachsen des Gelenkimplantats 12 an die Knochen 16 und 18 beziehungsweise die Wirbelkörper 22 und 24 oder andere Knochen des Patienten zu verbessern.

Das Gelenkimplantat 12 ist insgesamt einstückig ausgebildet. Es wird also vorzugsweise aus einem Stück hergestellt. Insbesondere kann dies durch spanende Bearbeitung eines massiven Körpers erfolgen oder beispielsweise durch Spritzgießen und/oder 3D-Druck. Diese Herstellungsverfahren eignen sich sowohl für Metalle als auch Kunststoffe. Beispielsweise ist es denkbar, das Gelenkimplantat 12 vollständig patientenindividuell auszubilden. Insbesondere ist es unter Einsatz verfügbarer 3D-Drucker grundsätzlich auch denkbar, das Gelenkimplantat 12 erst im Verlauf einer Operation herzustellen, wenn Knochenflächen für die Knochenanlageflächen 34 und/oder 36 bekannt oder präpariert sind. So ist keine Lagerhaltung für Gelenkimplantate 12 erforderlich und dem Patient kann das optimal für ihn abgestimmte und passende Gelenkimplantat 12 implantiert werden.

Die Implantation des beschriebenen einstückig ausgebildeten Gelenkimplantats 12 ist im Vergleich zum Einsatz bekannter Gelenkimplantate, die mehrteilig ausgebildet sind, deutlich weniger zeitaufwendig, da das Gelenkimplantat 12 als Ganzes in einem Stück eingesetzt werden kann.

Durch die Form der Profile der Festkörpergelenke 68 und 70 lässt sich eine Biege- beziehungsweise Torsionssteifigkeit des Gelenkimplantats 12 genau vorgeben. Man macht sich bei dem Gelenkimplantat 12 somit das Prinzip der Elastostatik zunutze. Die Funktion des Gelenkimplantats 12 wird somit insgesamt durch einen oder mehrere Bereiche verminderter Biege- beziehungsweise Torsionssteifigkeit relativ zu zwei oder mehreren angrenzenden Bereichen höherer Biege- beziehungsweise Torsionssteifigkeit erreicht.

Durch die Orientierung oder gegebenenfalls andere Formgebung der Festkörpergelenke 68 und 70 können sowohl isotrope als auch anisotrope Verformungseigenschaften für das Gelenkimplantat 12 vorgegeben werden.

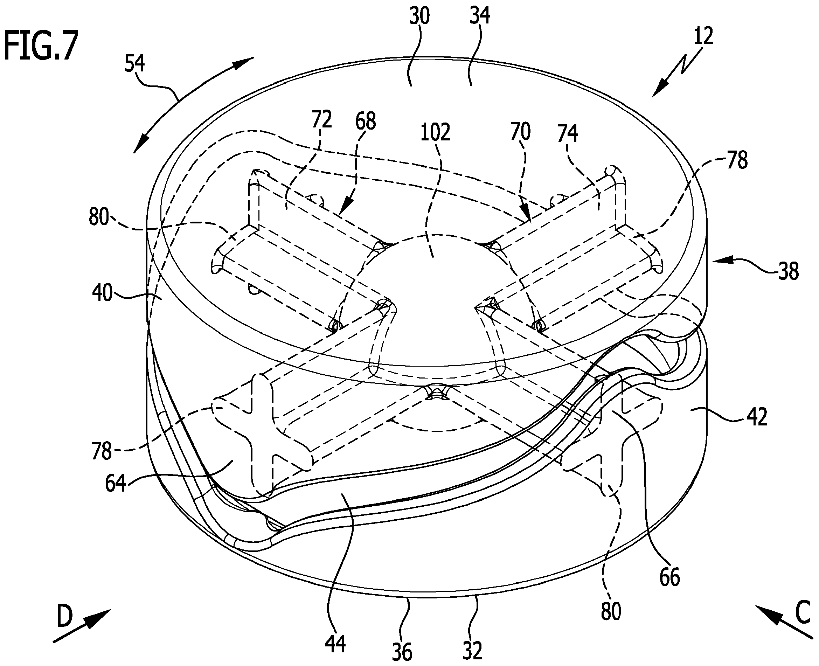

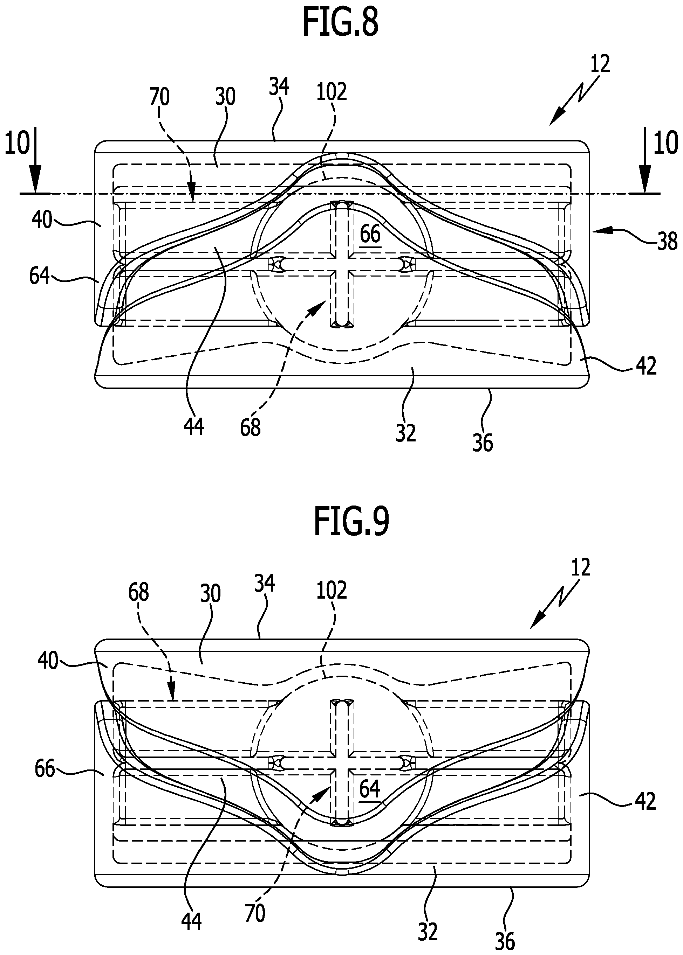

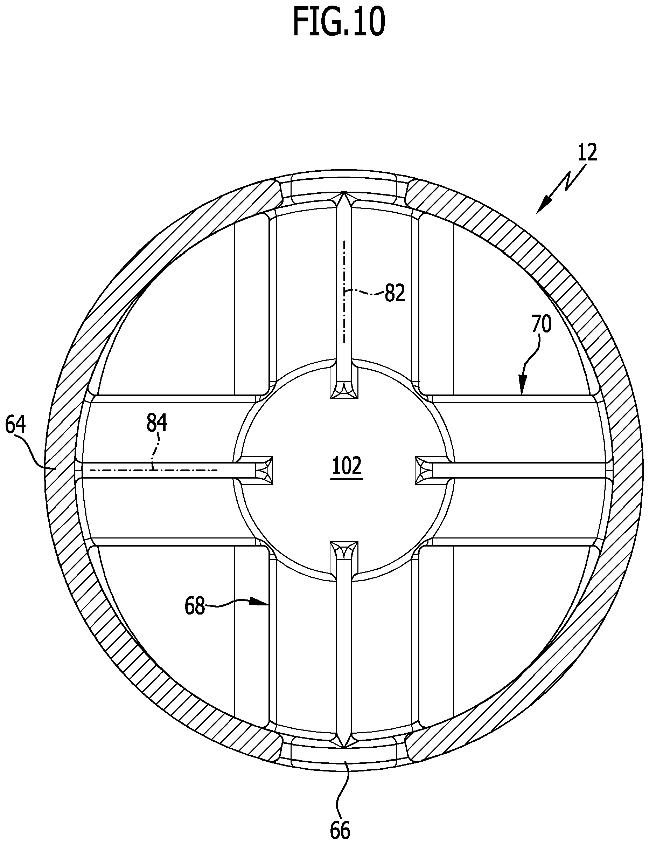

In den Figuren 7 bis 10 ist ein weiteres Ausführungsbeispiel eines Gelenkimplantats 12 schematisch dargestellt. Es stimmt im Aufbau mit dem in den Figuren 3 bis 6 dargestellten Gelenkimplantat im Wesentlichen überein. Der Übersichtlichkeit halber sind daher in den Figuren 7 bis 10 nur diejenigen Teile und Elemente des Gelenkimplantats 12 im Einzelnen bezeichnet, die zusätzlich vorhanden oder leicht modifiziert sind. Dies gilt entsprechend auch für die weiteren, nachfolgend noch im Detail beschriebenen Ausführungsbeispiele von Gelenkimplantaten 12 sowie Festkörpergelenken 68 und 70.

Der Kreuzungsbereich 76 ist bei dem in den Figuren 7 bis 10 dargestellten Gelenkimplantat 12 in Form eines Kreuzungskörpers 102 ausgebildet. Dieser ist kugelförmig gestaltet. Die Festkörpergelenke 68 und 70 erstrecken sich vom Kreuzungskörper 102 weg zu ihren freien Enden 78 beziehungswiese 80, so dass die Lagerelemente 64 und 66 jeweils über einen Abschnitt des Festkörpergelenks 68, den Kreuzungskörper 102 und einen Abschnitt des Festkörpergelenks 70 miteinander verbunden sind.

Der Kreuzungskörper 102 kann massiv oder auch in Form eines hohlen Körpers ausgebildet sein. Durch die Größe des Kreuzungskörpers 102 kann eine Steifigkeit der Festkörpergelenke 68 und 70 variiert werden.

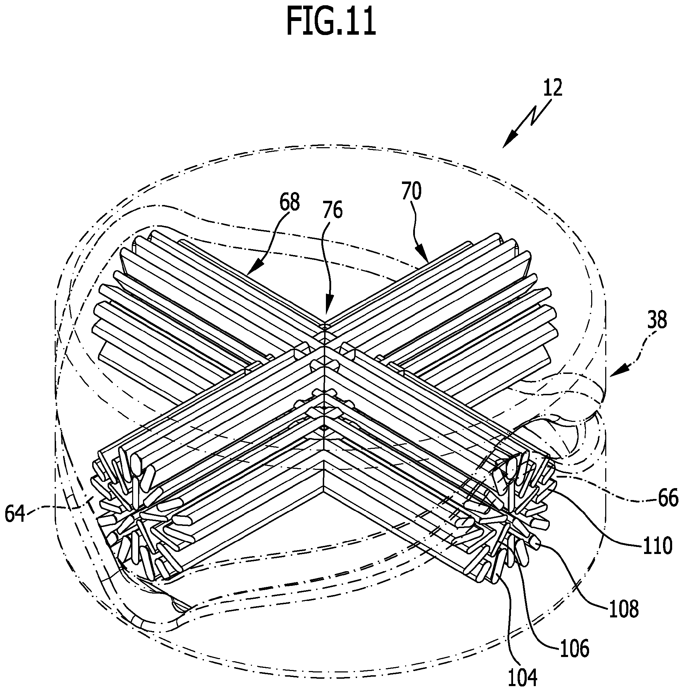

In den Figuren 11 bis 13 ist schematische eine alternative Ausgestaltung der beiden Festkörpergelenke 68 und 70 dargestellt. Sie unterscheiden sich von den Festkörpergelenken 68 und 70 des in den Figuren 3 bis 6 dargestellten Gelenkimplantats 12 dadurch, dass sie jeweils durch mehrere streifenförmige, sich bezogen auf die Längsachsen 82 und 84 in radialer Richtung weg weisend erstreckende Verbindungsstege 104, 106, 108 und 110 ausgebildet sind.

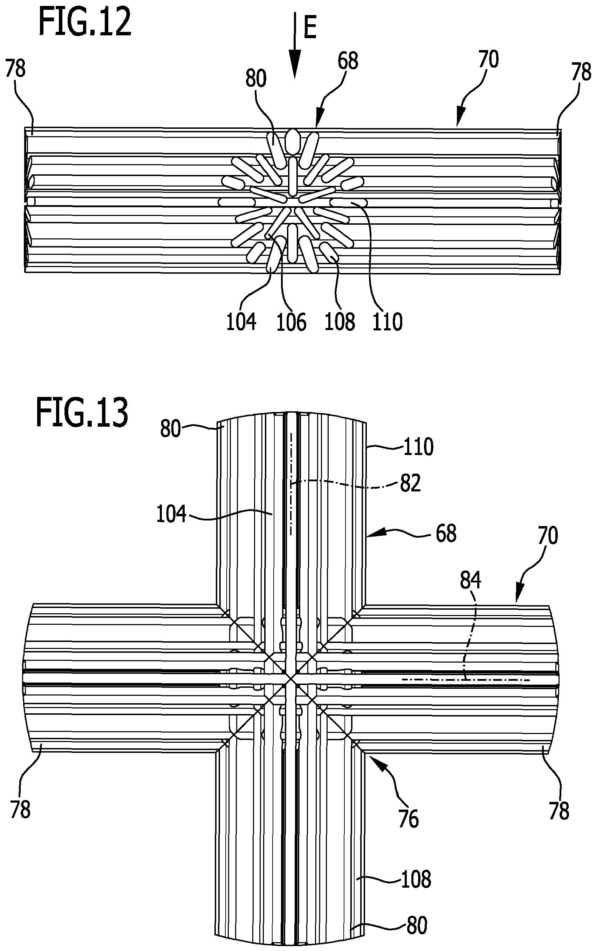

Die Verbindungsstege 104, 106, 108 und 110 unterscheiden sich sowohl in ihrer Länge als auch in ihrer Dicke und bilden, wie insbesondere in Figur 12 gut zu erkennen, im Querschnitt Radialstrahlen. Die Verbindungsstege 104, 106, 108 und 110 sind lediglich im Kreuzungsbereich 76 miteinander verbunden. Zwischen dem Kreuzungsbereich 76 und freien Enden 78 beziehungsweise 80 sind die Verbindungsstege 104, 106, 108 und 110 räumlich voneinander getrennt.

Im Gegensatz zu den Festkörpergelenken 68 und 70 des in den Figuren 3 bis 6 dargestellten Ausführungsbeispiels des Gelenkimplantats 12 umfassen diejenigen des in den Figuren 11 bis 13 dargestellten Gelenkimplantats 12 somit eine Mehrzahl an Verbindungsstegen 104, 106, 108 und 110. Durch die Gestaltung der Verbindungsstege 104, 106, 108 und 110, insbesondere deren Dicke sowie radiale Erstreckung, kann eine Torsionssteifigkeit der Festkörpergelenke 68 und 70 eingestellt werden.

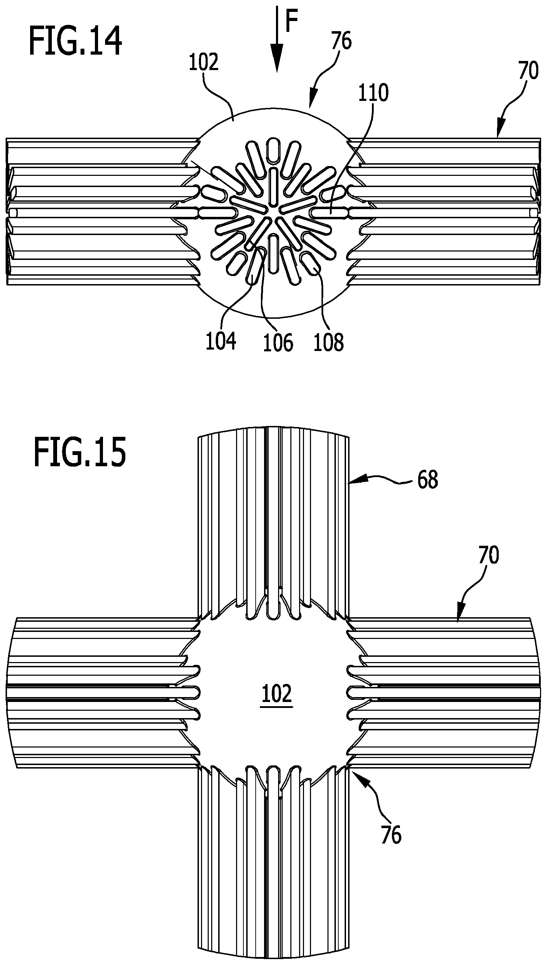

In den Figuren 14 und 15 ist eine weitere Alternative zweier Festkörpergelenke 68 und 70 schematisch dargestellt. Sie unterscheiden sich in ihrem Aufbau von den Festkörpergelenken 68 und 70 der Figuren 11 bis 13 dadurch, dass die Verbindungsstege 104, 106, 108 und 110 über den den Kreuzungsbereich 76 definierenden Kreuzungskörper 102, der im Wesentlichen kugelförmig ausgebildet ist, verbunden sind.

Die in den Figuren 14 und 15 dargestellten Festkörpergelenke 68 und 70 können wahlweise auch statt der Festkörpergelenke 68 und 70 bei den in den Figuren 3 bis 11 dargestellten Gelenkimplantaten 12 eingesetzt werden.

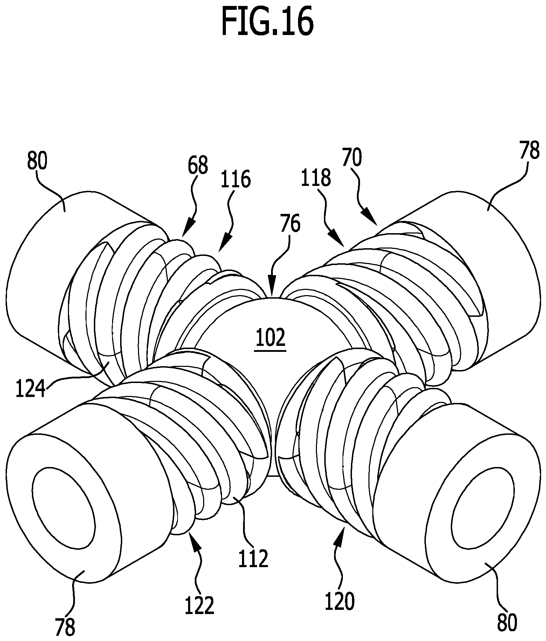

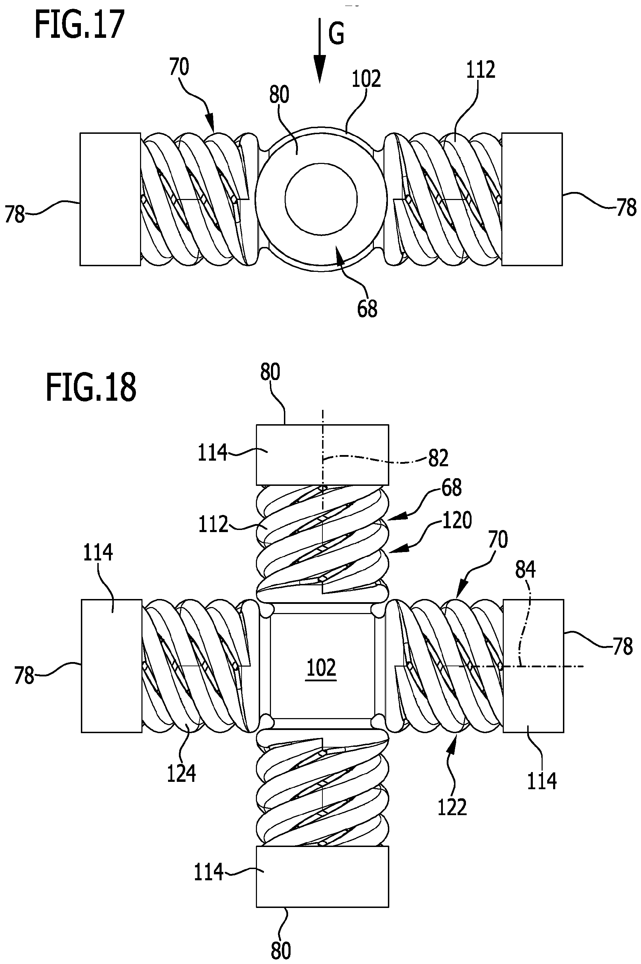

In den Figuren 16 bis 18 ist ein weiteres Ausführungsbeispiel für zwei miteinander zusammenwirkende Festkörpergelenke 68 und 70 beispielhaft dargestellt. Die Festkörpergelenke 68 und 70 sind jeweils durch mehrere, spiralförmig um die jeweiligen Längsachsen 82 beziehungsweise 84 gewundene Verbindungsstege 112 ausgebildet. Diese münden einerseits in den den Kreuzungsbereich 76 definierenden, im Wesentlichen kugelförmigen Kreuzungskörper 102 und andererseits in den hülsenförmigen Endringen 114, die die Enden 78 und 80 der Festkörpergelenke 68 und 70 definieren.

Die Verbindungsstege 112 ermöglichen sowohl eine Torsion der Festkörpergelenke 68 und 70 um deren Längsachsen 82 und 84 sowie auch eine Bewegung der Endringe 114 aufeinander zu und voneinander weg. Auf diese Weise wird eine Beweglichkeit der Knochenanlageelemente 30 und 32 zusätzlich erhöht, da diese, anders als bei dem in den Figuren 3 bis 6 dargestellten Ausführungsbeispiel, auch eine Bewegung der Knochenanlageelemente 30 und 32 in einer Ebene senkrecht zur Richtung 48 beziehungsweise zur Mittelachse 86 ermöglichen. Dies kann auch als eine Querverschiebung der Knochenanlageelemente 30 und 32 bezeichnet werden.

Durch die Anzahl der ineinander verschlungenen Verbindungsstege 112, beispielsweise können dies 2, 3, 4 oder mehr sein, lässt sich die Biege- und Torsionssteifigkeit der Festkörpergelenke 68 und 70 einstellen sowie auch deren Zug- und Druckfestigkeit.

Die Festkörpergelenke 68 und 70 umfassen somit Federelemente 116 und 118, die in Form von Schraubenfedern 120 beziehungswiese 122 ausgebildet sind. Die Verbindungsstege 112 bilden in sich verschlungene Federglieder.

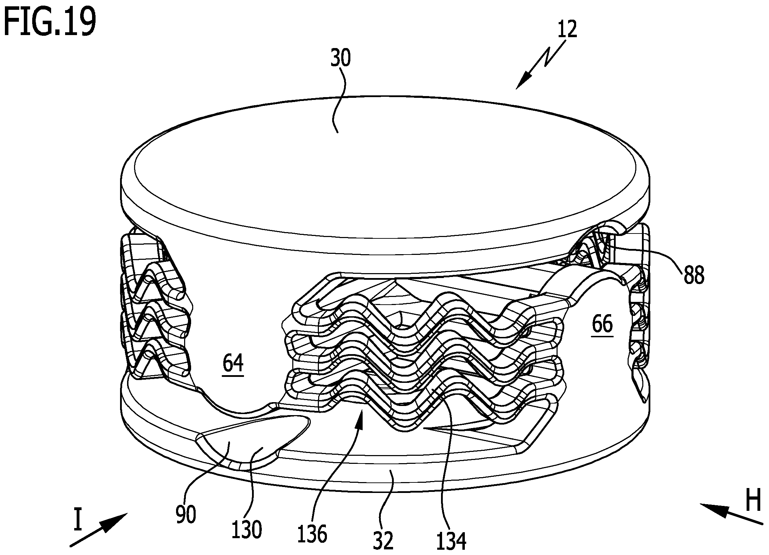

In den Figuren 19 bis 23 ist ein Ausführungsbeispiel eines erfindungsgemäßen Gelenkimplantats 12 schematisch dargestellt. Es ähnelt in seinem Aufbau dem in den Figuren 3 bis 6 dargestellten Gelenkimplantat. Allerdings unterscheidet es sich von diesem in der Ausgestaltung der Festkörpergelenke 68 und 70. Diese weisen einen sternförmigen Querschnitt mit acht sich radial von den Längsachsen 82 und 84 weg erstreckenden, identisch langen Verbindungsstegen 126 auf. Man kann auch sagen, die Festkörpergelenke 68 und 70 werden durch zwei um 45° um die jeweilige Längsachse 82 beziehungsweise 84 gedrehte kreuzförmige Profile gebildet.

Anders als beim Gelenkimplantat 12, das in den Figuren 3 bis 6 dargestellt ist, weist das Gelenkimplantat 12, das in den Figuren 19 bis 21 dargestellt ist, keine umlaufende Seitenwand auf. Vielmehr sind die Lagerelemente 64 und 66 in Form von von den Knochenanlageelementen 30 und 32 an deren äußeren Rändern abstehenden Vorsprüngen ausgebildet, deren freie Enden in Richtung auf das jeweils andere Knochenanlageelement 30 beziehungsweise 32 hin weisend konvex gekrümmt sind. Den Lagerelementen 64 und 66 gegenüberliegend angeordnet sind Führungsausnehmungen 88 und 90 in Form flacher Nuten 128 mit in Richtung auf das jeweils andere Knochenanlageelement 30 beziehungsweise 32 hin weisender konkaver Führungsfläche 130.

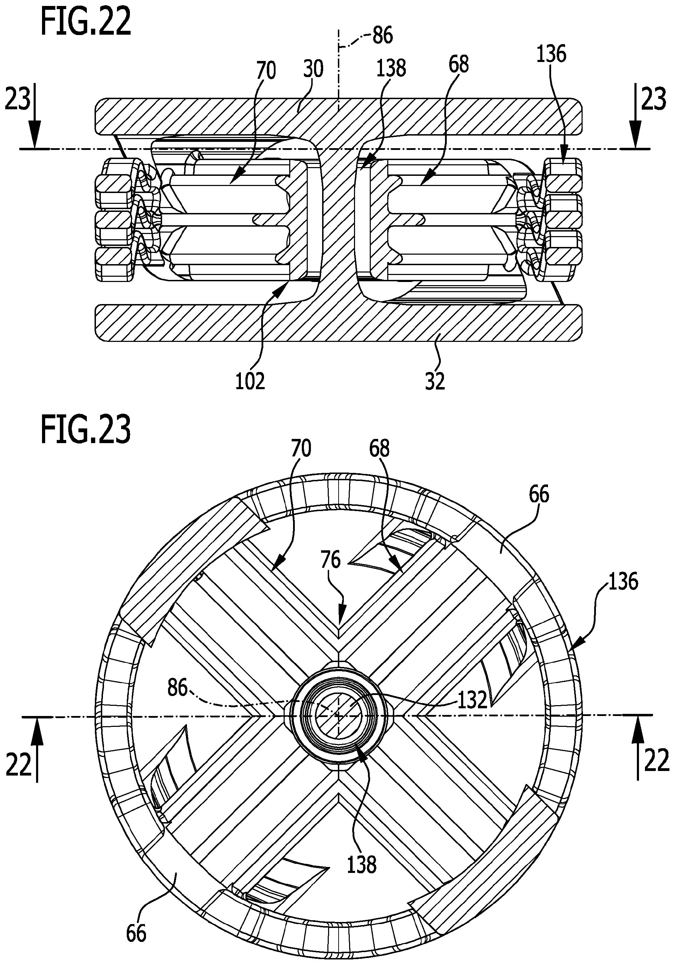

Wie in den Figuren 22 und 23 gut zu erkennen, sind die Festkörpergelenke 68 und 70 im Kreuzungsbereich 76 miteinander verbunden. Der Kreuzungsbereich 76 ist in Form eines hülsenförmigen Kreuzungskörpers ausgebildet. Dieser ist durchsetzt von einem die Mittelachse 86 definierenden, rotationssymmetrischen Verbindungsstab 132, der die beiden Knochenanlageelemente 30 und 32 direkt miteinander verbindet.

Der Verbindungsstab 132 bildet ein weiteres Festkörpergelenk 138 und ist bieg- und tordierbar. Zur Einstellung einer Biege- und Torsionssteifigkeit ist der Verbindungsstab ausgehend jeweils von den beiden Knochenanlageelementen 30 und 32 sich verjüngend ausgebildet, so dass er einen minimalen Querschnitt in etwa in der Mitte zwischen den beiden Knochenanlageelementen 30 und 32 aufweist.

Die Knochenanlageelemente 30 und 32 sind bei dem in den Figuren 19 bis 23 dargestellten Ausführungsbeispiel des Gelenkimplantats 12 nicht nur indirekt über die beiden im Bereich des Kreuzungskörpers 102 miteinander verbundenen Festkörpergelenke 68 und 70 miteinander verbunden, sondern zusätzlich durch mehrere wellenförmig in Umfangsrichtung verlaufend angeordnete Federglieder 134. Die Federglieder bilden jeweils als Dreiergruppen ein Federelement 136 mit freien Enden aus, wobei eines der beiden freien Enden an ein Lagerelement 64 und das andere an ein Lagerelement 66 angeformt ist. Damit verbinden die Federelemente 136 in Umfangsrichtung um 90° versetzt angeordnete Lagerelemente 64 des Knochenanlageelements 30 jeweils mit zwei Lagerelementen 66 des Knochenanlageelements 32 direkt miteinander.

Die Federglieder 134 begrenzen eine Verdrehung der Knochenanlageelemente 30 und 32 relativ zueinander um die Mittelachse 86. Im Übrigen sind die Knochenanlageelemente 30 und 32 auch wiederum gegeneinander verkippbar.

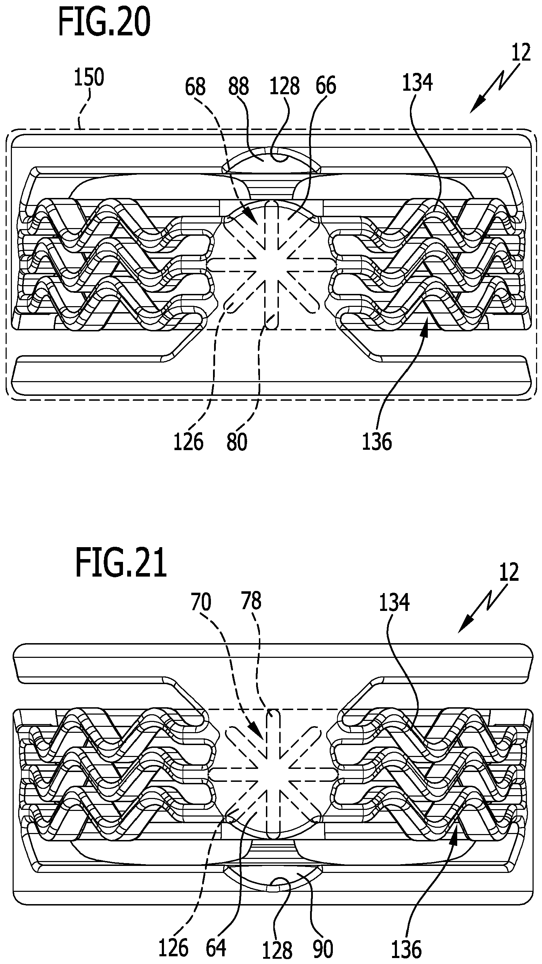

In Figur 20 ist ein weiteres Ausführungsbeispiel eines Gelenkimplantats 12 beispielhaft dargestellt. Es entspricht in seinem Aufbau im Wesentlichen dem in den Figuren 19 bis 23 dargestellten Gelenkimplantat 12, umfasst jedoch zusätzlich weitere, von der Mittelachse 86 weg weisend gekrümmte, Festkörpergelenke ausbildende Verbindungsstege 140. Diese sind jeweils in Gruppen zu dritt zwischen Abschnitten der Festkörpergelenke 68 und 70 angeordnet. Die Verbindungsstege 140 bilden sowohl verbieg- als auch tordierbare Federglieder, die eine Kippbewegung der Knochenanlageelemente 30 und 32, abhängig von der Wahl des Materials und den Abmessungen der insgesamt ausgebildeten Festkörpergelenke, relativ zueinander erschweren können.

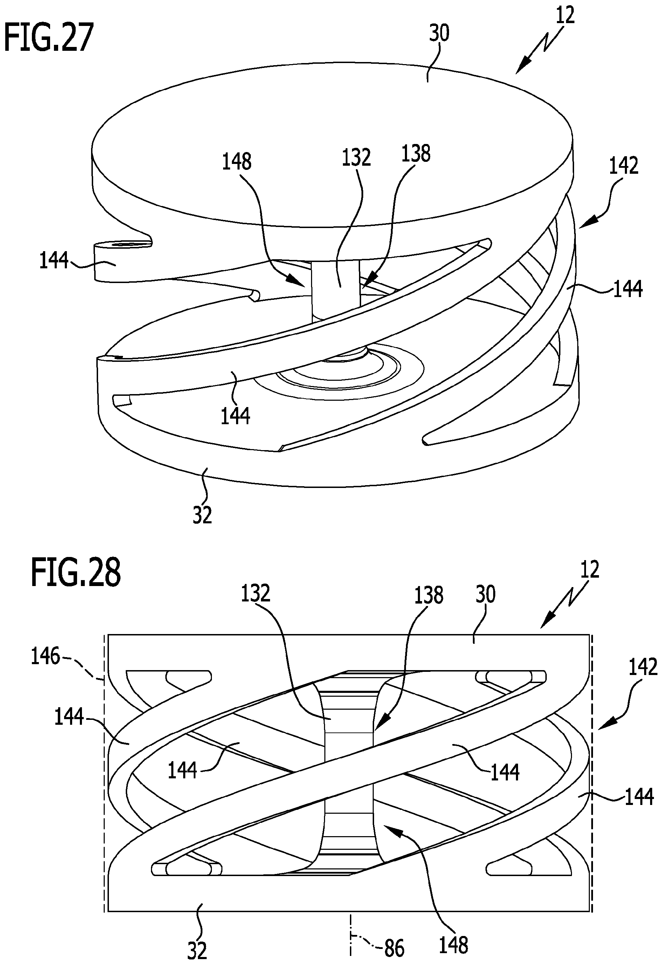

In den Figuren 27 und 28 ist ein weiteres Ausführungsbeispiel eines Gelenkimplantats 12 schematisch dargestellt. Es umfasst zwei kreisscheibenförmige Knochenanlageelemente 30 und 32, die über einen die Mittelachse 86 definierenden Verbindungsstab direkt miteinander verbunden sind.

Der Verbindungsstab 132 bildet ein erstes Festkörpergelenk 138.

Zusätzlich umfasst das Gelenkimplantat 12 vier weitere Festkörpergelenke 142 in Form von sich in Umfangsrichtung bezogen auf die Mittelachse 86 ausgehend von einem äußeren Randbereich der Knochenanlageelemente 30 und 32 als Ausschnitt einer Schraubenspirale erstreckenden Verbindungsstegen 144. Die insgesamt vier Verbindungsstege 144 erstrecken sich jeweils über einen Umfangswinkel von etwa 135°, sodass sie, ähnlich wie bei einem viergängigen Gewinde, vier in sich verschlungene Verbindungsstege 144 bilden. Die Festkörpergelenke 142 begrenzen sowohl eine Kippbewegung der Knochenanlageelemente 30 und 32 aufeinander zu sowie voneinander weg als auch eine Verdrehbewegung der Knochenanlageelemente 30 und 32 um die Mittelachse 86.

Die Materialien, aus denen alle oben beschriebenen Gelenkimplantate 12 ausgebildet werden können, wurden bereits oben nicht abschließend genannt.

Des Weiteren kann optional bei allen beschriebenen Gelenkimplantaten eine Hülle 146 vorgesehen sein, die elastisch und/oder flexibel ausgebildet ist und einen zwischen den Knochenanlageelementen 30 und 32 definierten Gelenkraum 148 verschließt. Die Hülle 146 kann, wie schematisch in Figur 28 dargestellt, im Wesentlichen hülsenförmig ausgebildet sein. Sie kann jedoch aber auch, wie beispielhaft gestrichelt in Figur 20 dargestellt, das Gelenkimplantat 12 in Form einer Gelenkhülle 150 vollständig umgeben, also insbesondere auch die Knochenanlageelemente 30 und 32. So lässt sich insbesondere verhindern, dass Weichgewebe oder Knochen in den Gelenkraum 148 eindringen oder einwachsen können.

Des Weiteren kann der Gelenkraum 148, insbesondere dann, wenn eine Hülle 146 oder eine Gelenkhülle 150 vorgesehen sind, auch mit einem Füllmaterial befüllt werden. Beispielsweise kann es sich bei dem Füllmaterial um ein Fluid, insbesondere eine Flüssigkeit handeln. Die Flüssigkeit kann insbesondere Wasser oder eine isotonische Kochsalzlösung sein. Die Hülle 146 und/oder die Gelenkhülle 150 können insbesondere fluiddicht oder auch als permeable oder semipermeable Membran ausgebildet sein

Alternativ kann das Füllmaterial auch in Form von in den Gelenkraum 148 eingebrachten elastischen Elementen, wie beispielsweise Kunststoffkügelchen, ausgebildet sein. Denkbar ist es auch, als Füllmaterial Silikon oder dergleichen zu wählen. Auch Gelatine oder andere gallertartige und körperverträgliche Materialien können optional eingesetzt werden.

Die Form der Knochenanlageelemente 30 und 32 kann selbstverständlich auch von der Form einer Kreisscheibe abweichen. Insbesondere sind nierenförmige Gestaltungen möglich und denkbar. Entsprechend können auch die Festkörpergelenke nicht symmetrisch angeordnet sein, so dass sich insgesamt anisotrope Biege- und Torsionseigenschaften des Gelenkimplantats ergeben und gezielt einstellen lassen.

Idealerweise ist die jeweilige Knochenanlagefläche des Knochenanlageelements an eine Form des Knochens angepasst, an dem das Knochenanlageelement 30 beziehungsweise 32 festgelegt werden soll.

In den Figuren nicht dargestellt, jedoch optional auch möglich, sind ferner Verankerungselemente, die von den Knochenanlageflächen 34 und/oder 36 abstehend ausgebildet sind, um in entsprechenden Kavitäten der Knochen eingebracht zu werden oder schlicht zum Verankern des Gelenkimplantats am Knochen dienen. Sie können spitz, also insbesondere nagel- oder pinartig, oder auch stumpf, also insbesondere in Form von zylindrischen Stiften, ausgebildet sein. Auf diese Weise kann das Gelenkimplantat 12 idealerweise ohne Verwendung von Knochenzement an einem Knochen festgelegt werden.

Die Ausbildung der Knochenanlageelemente 30 und 32 mit patientenindividuellen Knochenanlageflächen 34 bis 36 lässt sich mittels 3D-Druck auf besonderes einfache Weise realisieren.

Ferner lassen sich die beschriebenen Festkörpergelenke der Gelenkimplantate 12 nahezu beliebig miteinander kombinieren, um so Eigenschaften der Gelenkimplantate 12 in gewünschter Weise einstellen zu können, insbesondere eine Verdreh- und/oder Verkippbarkeit der Knochenanlageelemente 30 und 32 relativ zueinander um eine oder mehrere Achsen mit identischen oder unterschiedlichen Biege- und/oder Torsionssteifigkeiten.

### Bezugszeichenliste

- 10: Fußskelett
- 12: Gelenkimplantat
- 14: Sprunggelenkimplantat
- 16: erster Knochen
- 18: zweiter Knochen
- 20: Zwischenwirbelimplantat
- 22: Wirbelkörper
- 24: Wirbelkörper
- 26: Wirbelsäule
- 28: Bandscheibe
- 30: Knochenanlageelement
- 32: Knochenanlageelement
- 34: Knochenanlagefläche
- 36: Knochenanlagefläche
- 38: Seitenwand
- 40: erster Seitenwandteil
- 42: zweiter Seitenwandteil
- 44: Schlitz
- 46: Abstand
- 48: Richtung
- 50: Höhe
- 52: Höhe
- 54: Umfangsrichtung
- 56: Minimum
- 58: Maximum
- 60: Maximum
- 62: Minimum
- 64: Lagerelement
- 66: Lagerelement
- 68: Festkörpergelenk
- 70: Festkörpergelenk
- 72: Verbindungssteg
- 74: Verbindungssteg
- 76: Kreuzungsbereich
- 78: Ende
- 80: Ende
- 82: Längsachse
- 84: Längsachse
- 86: Mittelachse
- 88: Führungsausnehmung
- 90: Führungsausnehmung
- 92: Randfläche
- 94: Randfläche
- 96: Anschlagsfläche
- 98: Anschlagfläche
- 100: Winkel
- 102: Kreuzungskörper
- 104: Verbindungssteg
- 106: Verbindungssteg
- 108: Verbindungssteg
- 110: Verbindungssteg
- 112: Verbindungssteg
- 114: Endring
- 116: Federelement
- 118: Federelement
- 120: Schraubenfeder
- 122: Schraubenfeder
- 124: Federglied
- 126: Verbindungssteg
- 128: Nut
- 130: Führungsfläche
- 132: Verbindungsstab
- 134: Federglied
- 136: Federelement
- 138: Festkörpergelenk
- 140: Verbindungssteg
- 142: Festkörpergelenk
- 146: Hülle
- 148: Gelenkraum
- 150: Gelenkhülle

## Patentansprüche

1. Künstliches Gelenkimplantat (12) mit einem ersten, an einem ersten Knochen (16; 22) oder Knochenteil anleg- oder festlegbaren Knochenanlageelement (30) und einem zweiten, an einem zweiten Knochen (18; 24) oder Knochenteil anleg- oder festlegbaren Knochenanlageelement (32), wobei das erste und das zweite Knochenanlageelement (30, 32) relativ zueinander bewegbar angeordnet und gelenkig miteinander gekoppelt sind, wobei das Gelenkimplantat (12) einstückig ausgebildet ist, wobei das Gelenkimplantat (12) mindestens ein Festkörpergelenk (68, 70, 138, 142) umfasst, welches das erste und das zweite Knochenanlageelement (30, 32) gelenkig miteinander verbindet, wobei das mindestens eine Festkörpergelenk (68, 70, 138, 142) mindestens ein Federelement (116, 118, 136) umfasst, **dadurch gekennzeichnet, dass** das mindestens eine Federelement (136) zwei oder mehr, äquidistant oder im Wesentlichen äquidistant voneinander angeordnete, wellenförmig gewundene Federglieder (134) umfasst, welche zwei oder mehr Federglieder (134) in Umfangsrichtung der platten- oder scheibenförmigen Knochenanlageelemente (30, 32) angeordnet sind, um Lagerelemente (64, 66) der beiden Knochenanlageelemente (30, 32) miteinander zu verbinden.

2. Gelenkimplantat nach Anspruch 1, **dadurch gekennzeichnet, dass**
a) das mindestens eine Festkörpergelenk (68, 70, 138, 142) mindestens einen elastisch biegbaren Verbindungssteg (72, 74, 104, 106, 108, 110, 112, 140, 144) umfasst oder durch einen solchen ausgebildet ist
und/oder
b) das Gelenkimplantat zwei oder mehr Festkörpergelenke (68, 70, 138, 142) umfasst,
und/oder
c) das mindestens eine Festkörpergelenk (68, 70, 138, 142) in Form eines Torsionselements ausgebildet ist.

3. Gelenkimplantat nach einem der voranstehenden Ansprüche, **gekennzeichnet durch** mindestens zwei sich kreuzende Festkörpergelenke (68, 70, 138, 142).

4. Gelenkimplantat nach Anspruch 3, **dadurch gekennzeichnet, dass**
a) sich die mindestens zwei Festkörpergelenke (68, 70, 138, 142) in einem Kreuzungsbereich (76) kreuzen oder durchdringen oder dass die mindestens zwei Festkörpergelenke (68, 70, 138, 142) in einem Kreuzungsbereich (76) miteinander verbunden sind,
wobei insbesondere der Kreuzungsbereich (76) in Form eines Kreuzungskörpers (102) ausgebildet ist,
wobei weiter insbesondere der Kreuzungskörper (102) kugelförmig oder im Wesentlichen kugelförmig ausgebildet ist,
und/oder
b) die mindestens zwei Festkörpergelenke (68, 70, 138, 142) einen unterschiedlichen Querschnitt aufweisen, wobei die mindestens zwei Festkörpergelenke (68, 70, 138, 142) insbesondere unterschiedliche Elastizitäten in unterschiedlichen Raumrichtungen aufweisen.

5. Gelenkimplantat nach einem der voranstehenden Ansprüche, **dadurch gekennzeichnet, dass**
a) das erste Knochenanlageelement (30) eine ebene oder im Wesentlichen ebene Knochenanlagefläche (34) aufweist und/oder dass das zweite Knochenanlageelement (32) eine ebene oder im Wesentlichen ebene Knochenanlagefläche (36) aufweist,
wobei insbesondere mindestens eine der beiden Knochenanlageflächen (34, 36) mit einer osteointegrativen Beschichtung versehen ist
und/oder
sich das mindestens eine Festkörpergelenk (68, 70) parallel oder im Wesentlichen parallel zu einer Knochenanlagefläche (34, 36) erstreckt,
und/oder
b) das mindestens eine Festkörpergelenk (142) das erste und das zweite Knochenanlageelement (30, 32) spiralförmig miteinander verbindet.

6. Gelenkimplantat nach einem der voranstehenden Ansprüche, **dadurch gekennzeichnet, dass** das erste Knochenanlageelement (30) mindestens zwei erste Lagerelemente (64) für freie Enden eines ersten Festkörpergelenks (68, 70) aufweist, dass das zweite Knochenanlageelement (32) mindestens zwei zweite Lagerelemente (66) für freie Enden eines zweiten Festkörpergelenks (68, 70) aufweist und dass sich das erste und das zweite Festkörpergelenk (68, 70) kreuzen oder durchdringen oder in einem Kreuzungsbereich (76) miteinander verbunden sind.

7. Gelenkimplantat nach Anspruch 6, **dadurch gekennzeichnet, dass**
a) die ersten und/oder zweiten Lagerelemente (64, 66) in Form von vom ersten oder zweiten Knochenanlageelement (30, 32) abstehenden Vorsprüngen ausgebildet sind
und/oder
b) die ersten und zweiten Lagerelemente (64, 66) jeweils einen Teil einer mindestens abschnittsweise umlaufenden Seitenwand (38, 40, 42) des ersten Knochenanlageelements (30) oder des zweiten Knochenanlageelements (32) bilden und dass die Seitenwände (38, 40, 42) des ersten und zweiten Knochenanlageelements (30, 32) gemeinsam eine durch einen umlaufenden Schlitz (44) getrennte Hülse definieren,
wobei insbesondere ein Abstand (50, 52) des Schlitzes (44) vom ersten Knochenanlageelement (30) und/oder vom zweiten Knochenanlageelement (32) in Abhängigkeit eines Umfangswinkels variiert
und/oder
den Schlitz (44) begrenzende Randflächen (92, 94) der Seitenwände (40, 42) Anschlagflächen (96, 98) zur Begrenzung einer Relativbewegung der ersten und zweiten Knochenanlageelemente (30, 32) definieren,
und/oder
c) jedes Lagerelement (64, 66) mit mindestens zwei weiteren Lagerelementen (64, 66) über Festkörpergelenke (68, 70) verbunden ist
und/oder
d) das erste und/oder das zweite Knochenanlageelement (30, 32) mindestens eine Führungsausnehmung (88, 90) für ein korrespondierendes Lagerelement (64, 66) des jeweils anderen Knochenanlageelements (30, 32) umfasst,
wobei insbesondere
d1) die mindestens eine Führungsausnehmung (88, 90) in Form einer Nut (128) ausgebildet ist
und/oder
d2) die mindestens eine Führungsausnehmung (88, 90) in Richtung auf das Lagerelement (64, 66) des anderen Knochenanlageelements (30, 32) hin weisend konkav gekrümmt ist, wobei insbesondere das mindestens eine Federelement (116, 118) in Form einer Schraubenfeder (120, 122) oder in Form einer Torsionsfeder ausgebildet ist
und/oder
das mindestens eine Federelement (116, 118, 136) zwei oder mehr, insbesondere in sich verschlungene, Federglieder (124, 134) umfasst.

8. Gelenkimplantat nach einem der voranstehenden Ansprüche, **dadurch gekennzeichnet, dass** das mindestens eine Festkörpergelenk (138) einen Verbindungsstab (132) umfasst.

9. Gelenkimplantat nach Anspruch 8, **dadurch gekennzeichnet, dass**
a) der Verbindungsstab (132) quer, insbesondere senkrecht, vom ersten und/oder zweiten Knochenanlageelement (30, 32) abstehend ausgebildet ist
und/oder
b) sich der Verbindungsstab (132) ausgehend vom ersten und/oder zweiten Knochenanlageelement (30, 32) verjüngt.

10. Gelenkimplantat nach einem der voranstehenden Ansprüche, **dadurch gekennzeichnet, dass**
a) das erste und/oder das zweite Knochenanlageelement (30, 32) in Form einer flachen Scheibe ausgebildet ist
und/oder
b) die mindestens zwei, insbesondere die sich kreuzenden, Festkörpergelenke (68, 70) einen identischen Querschnitt aufweisen
und/oder
c) ein Querschnitt des mindestens einen Festkörpergelenks (68, 70, 138, 142) kreuzförmig, sternförmig, vieleckig, rund oder oval ist.

11. Gelenkimplantat nach einem der voranstehenden Ansprüche, **dadurch gekennzeichnet, dass** zwischen dem ersten und dem zweiten Knochenanlageelement (30, 32) ein Füllmaterial eingebracht ist wobei insbesondere das Füllmaterial
a) elastisch ist
und/oder
b) ein Kunststoff ist, insbesondere Silikon.

12. Gelenkimplantat nach einem der voranstehenden Ansprüche, **dadurch gekennzeichnet, dass** das mindestens eine Festkörpergelenk (68, 70, 138, 142) von einer Hülle (146) oder von einer Kapsel umgeben ist, wobei insbesondere die Hülle (146) oder die Kapsel und/oder die Gelenkhülle (150) oder die Gelenkkapsel mit einem Fluid gefüllt sind, insbesondere mit einer körperverträglichen Flüssigkeit.

13. Gelenkimplantat nach einem der voranstehenden Ansprüche, **dadurch gekennzeichnet, dass** das Gelenkimplantat eine äußere Gelenkhülle (150) oder Gelenkkapsel umfasst,
wobei insbesondere das erste Knochenanlageelement (30) mindestens zwei erste Lagerelemente (64) für freie Enden eines ersten Festkörpergelenks (68, 70) aufweist, dass das zweite Knochenanlageelement (32) mindestens zwei zweite Lagerelemente (66) für freie Enden eines zweiten Festkörpergelenks (68, 70) aufweist und dass sich das erste und das zweite Festkörpergelenk (68, 70) kreuzen oder durchdringen oder in einem Kreuzungsbereich (76) miteinander verbunden sind, dass die ersten und zweiten Lagerelemente (64, 66) jeweils einen Teil einer mindestens abschnittsweise umlaufenden Seitenwand (38, 40, 42) des ersten Knochenanlageelements (30) oder des zweiten Knochenanlageelements (32) bilden, dass die Seitenwände (38, 40, 42) des ersten und zweiten Knochenanlageelements (30, 32) gemeinsam eine durch einen umlaufenden Schlitz (44) getrennte Hülse definieren und dass die Gelenkhülle (150) oder Gelenkkapsel den umlaufenden Schlitz (44) verschließt.

14. Gelenkimplantat nach einem der voranstehenden Ansprüche, **dadurch gekennzeichnet, dass** das Gelenkimplantat (12) aus einem implantierbaren Metall oder Kunststoff hergestellt ist,
wobei insbesondere das Metall ein Implantatstahl, insbesondere ein Kobalt-Chrom-Stahl, oder Titan ist oder enthält und dass der Kunststoff Polyetheretherketon (PEEK), Polyethylen (PE) und/oder Silikon ist oder enthält.

15. Gelenkimplantat nach einem der voranstehenden Ansprüche, **dadurch gekennzeichnet, dass** das Gelenkimplantat (12)
a) durch Spritzgießen und/oder 3D-Druck hergestellt ist und/oder
b) in Form eines künstlichen Sprunggelenks (14), eines Zwischenwirbelimplantats (20), eines Hüftgelenks, eines Kniegelenks, eines Schultergelenks, eines Finger- oder Zehengelenks ausgebildet ist.

## Claims

1. Artificial joint implant (12) having a first bone abutment element (30), which is placeable or fixable on a first bone (16; 22) or bone part, and a second bone abutment element (32), which is placeable or fixable on a second bone (18; 24) or bone part, wherein the first and the second bone abutment element (30, 32) are arranged so as to be moveable relative to each other and are articulatedly coupled to each other, wherein the joint implant (12) is formed in one piece, wherein the joint implant (12) comprises at least one flexure joint (68, 70, 138, 142) which articulatedly connects the first and the second bone abutment element (30, 32) to each other, wherein the at least one flexure joint (68, 70, 138, 142) comprises at least one spring element (116, 118, 136), **characterized in that** the at least one spring element (136) comprises two or more spring members (134) which are wound in an undulating manner and are arranged equidistantly or substantially equidistantly from each other, which two or more spring members (134) are arranged in the circumferential direction of the plate- or disc-shaped bone abutment elements (30, 32) in order to connect bearing elements (64, 66) of the two bone abutment elements (30, 32) to each other.

2. Joint implant in accordance with Claim 1, **characterized in that**
a) the at least one flexure joint (68, 70, 138, 142) comprises or is formed by at least one elastically bendable connecting web (72, 74, 104, 106, 108, 110, 112, 140, 144)
and/or
b) the joint implant comprises two or more flexure joints (68, 70, 138, 142),
and/or
c) the at least one flexure joint (68, 70, 138, 142) is configured in the form of a torsional element.

3. Joint implant in accordance with any one of the preceding Claims, **characterized by** at least two intersecting flexure joints (68, 70, 138, 142).

4. Joint implant in accordance with Claim 3, **characterized in that**
a) the at least two flexure joints (68, 70, 138, 142) intersect or interpenetrate in an intersection region (76), or **in that** the at least two flexure joints (68, 70, 138, 142) are connected to each other in an intersection region (76),
wherein in particular the intersection region (76) is configured in the form of an intersection body (102),
wherein further particularly, the intersection body (102) is of spherical or substantially spherical configuration,
and/or
b) the at least two flexure joints (68, 70, 138, 142) have a different cross section, wherein the at least two flexure joints (68, 70, 138, 142) have, in particular, different elasticities in different spatial directions.

5. Joint implant in accordance with any one of the preceding Claims, **characterized in that**
a) the first bone abutment element (30) has a planar or substantially planar bone abutment face (34) and/or **in that** the second bone abutment element (32) has a planar or substantially planar bone abutment face (36),
wherein in particular at least one of the two bone abutment faces (34, 36) is provided with an osteointegrative coating and/or
the at least one flexure joint (68, 70) extends in parallel or substantially in parallel to a bone abutment face (34, 36),
and/or
b) the at least one flexure joint (142) connects the first and the second bone abutment element (30, 32) to each other in the form of a spiral.

6. Joint implant in accordance with any one of the preceding Claims, **characterized in that** the first bone abutment element (30) has at least two first bearing elements (64) for free ends of a first flexure joint (68, 70), **in that** the second bone abutment element (32) has at least two second bearing elements (66) for free ends of a second flexure joint (68, 70) and **in that** the first and the second flexure joint (68, 70) intersect or interpenetrate or are connected to each other in an intersection region (76).

7. Joint implant in accordance with Claim 6, **characterized in that**
a) the first and/or second bearing elements (64, 66) are configured in the form of projections protruding from the first or second bone abutment element (30, 32)
and/or
b) the first and second bearing elements (64, 66) each form a part of a side wall (38, 40, 42) of the first bone abutment element (30) or the second bone abutment element (32), which side wall (38, 40, 42) is circumferential at least in sections, and **in that** the side walls (38, 40, 42) of the first and second bone abutment element (30, 32) together define a sleeve separated by a circumferential slot (44),
wherein in particular a distance (50, 52) of the slot (44) from the first bone abutment element (30) and/or from the second bone abutment element (32) varies in dependence on a circumferential angle
and/or
rim faces (92, 94) of the side walls (40, 42) which delimit the slot (44) define stop faces (96, 98) for delimiting a relative movement of the first and second bone abutment elements (30, 32),
and/or
c) each bearing element (64, 66) is connected to at least two further bearing elements (64, 66) by way of flexure joints (68, 70)
and/or
d) the first and/or the second bone abutment element (30, 32) comprises at least one guide recess (88, 90) for a corresponding bearing element (64, 66) of the respective other bone abutment element (30, 32),
wherein in particular
d1) the at least one guide recess (88, 90) is configured in the form of groove (128)
and/or
d2) the at least one guide recess (88, 90) is concavely curved pointing in the direction toward the bearing element (64, 66) of the other bone abutment element (30, 32),
wherein in particular the at least one spring element (116, 118) is configured in the form of a coil spring (120, 122) or in the form of a torsional spring
and/or
the at least one spring element (116, 118, 136) comprises two or more, in particular interwoven, spring members (124, 134).

8. Joint implant in accordance with any one of the preceding Claims, **characterized in that** the at least one flexure joint (138) comprises a connecting rod (132).

9. Joint implant in accordance with Claim 8, **characterized in that**
a) the connecting rod (132) is configured protruding transversely, in particular perpendicularly, from the first and/or second bone abutment element (30, 32)
and/or
b) the connecting rod (132) tapers commencing from the first and/or second bone abutment element (30, 32).

10. Joint implant in accordance with any one of the preceding Claims, **characterized in that**
a) the first and/or the second bone abutment element (30, 32) is configured in the form of a flat disc
and/or
b) the at least two, in particular the intersecting, flexure joints (68, 70) have an identical cross section
and/or
c) a cross section of the at least one flexure joint (68, 70, 138, 142) is cross-shaped, star-shaped, polygonal, round, or oval.

11. Joint implant in accordance with any one of the preceding Claims, **characterized in that** a filling material is introduced between the first and the second bone abutment element (30, 32)
wherein in particular the filling material
a) is elastic
and/or
b) is a plastic, in particular silicone.

12. Joint implant in accordance with any one of the preceding Claims, **characterized in that** the at least one flexure joint (68, 70, 138, 142) is surrounded by a casing (146) or by a capsule,
wherein in particular the casing (146) or the capsule and/or the joint casing (150) or the joint capsule are filled with a fluid, in particular with a biocompatible liquid.

13. Joint implant in accordance with any one of the preceding Claims, **characterized in that** the joint implant comprises an outer joint casing (150) or joint capsule,
wherein in particular the first bone abutment element (30) has at least two first bearing elements (64) for free ends of a first flexure joint (68, 70), **in that** the second bone abutment element (32) has at least two second bearing elements (66) for free ends of a second flexure joint (68, 70), and **in that** the first and the second flexure joint (68, 70) intersect or interpenetrate or are connected to each other in an intersection region (76), **in that** the first and second bearing elements (64, 66) each form a part of a side wall (38, 40, 42) of the first bone abutment element (30) or the second bone abutment element (32), which side wall (38, 40, 42) is circumferential at least in sections, **in that** the side walls (38, 40, 42) of the first and second bone abutment element (30, 32) together define a sleeve separated by a circumferential slot (44), and **in that** the joint casing (150) or joint capsule closes the circumferential slot (44).

14. Joint implant in accordance with any one of the preceding Claims, **characterized in that** the joint implant (12) is made of an implantable metal or plastic,
wherein in particular the metal is or contains an implant steel, in particular a cobalt chrome steel, or titanium, and **in that** the plastic is or contains polyetheretherketone (PEEK), polyethylene (PE), and/or silicone.

15. Joint implant in accordance with any one of the preceding Claims, **characterized in that** the joint implant (12)
a) is produced by injection molding and/or 3D printing
and/or
b) is configured in the form of an artificial ankle joint (14), an intervertebral implant (20), a hip joint, a knee joint, a shoulder joint, a finger or toe joint.

## Revendications

1. Implant d'articulation artificiel (12) avec un premier élément d'appui d'os (30) pouvant être appuyé ou fixé sur un premier os (16 ; 22) ou partie d'os et un second élément d'appui d'os (32) pouvant être appuyé ou fixé sur un second os (18 ; 24) ou partie d'os, où le premier et le second élément d'appui d'os (30, 32) sont disposés de manière mobile l'un par rapport à l'autre et sont couplés l'un avec l'autre de manière articulée, où l'implant d'articulation (12) est formé d'une pièce, où l'implant d'articulation (12) comprend au moins une articulation solide (68, 70, 138, 142) qui relie l'un à l'autre le premier et le second élément d'appui d'os (30, 32) de manière articulée, où la au moins une articulation solide (68, 70, 138, 142) comprend au moins un élément de ressort (116, 118, 136), **caractérisé en ce que** le au moins un élément de ressort (136) comprend deux ou plusieurs membres de ressort (134) courbés de manière ondulée, disposés de manière équidistante ou sensiblement équidistante les uns des autres, lesquels deux ou plusieurs membres de ressort (134) sont disposés en direction de la périphérie des éléments d'appui d'os (30, 32) en forme de plaques ou de disques, pour relier entre eux des éléments de support (64, 66) des deux éléments d'appui d'os (30, 32).

2. Implant d'articulation selon la revendication 1, **caractérisé en ce que**
a) la au moins une articulation solide (68, 70, 138, 142) comprend au moins une traverse de liaison (72, 74, 104, 106, 108, 110, 112, 140, 144) flexible élastiquement ou est formée par une telle traverse de liaison
et/ou
b) l'implant d'articulation comprend deux ou plusieurs articulations solides (68, 70, 138, 142),
et/ou
c) la au moins une articulation solide (68, 70, 138, 142) est formée sous forme d'un élément de torsion.

3. Implant d'articulation selon l'une des revendications précédentes, **caractérisé par** au moins deux articulations solides (68, 70, 138, 142) qui se croisent.

4. Implant d'articulation selon la revendication 3, **caractérisé en ce que**
a) les au moins deux articulations solides (68, 70, 138, 142) se croisent ou se pénètrent dans un domaine de croisement (76) ou **en ce que** les au moins deux articulations solides (68, 70, 138, 142) sont reliées l'une à l'autre dans un domaine de croisement (76),
où en particulier le domaine de croisement (76) est formé sous forme d'un corps de croisement (102),
où en particulier encore le corps de croisement (102) est formé en forme de sphère ou sensiblement en forme de sphère,
et/ou
b) les au moins deux articulations solides (68, 70, 138, 142) présentent une section transversale différente, où les au moins deux articulations solides (68, 70, 138, 142) présentent en particulier des élasticités différentes dans des directions spatiales différentes.

5. Implant d'articulation selon l'une des revendications précédentes, **caractérisé en ce que**
a) le premier élément d'appui d'os (30) présente une surface d'appui d'os (34) plane ou sensiblement plane et/ou **en ce que** le second élément d'appui d'os (32) présente une surface d'appui d'os (36) plane ou sensiblement plane,
où en particulier au moins l'une des deux surfaces d'appui d'os (34, 36) est munie d'un revêtement ostéointégratif et/ou
la au moins une articulation solide (68, 70) s'étend parallèlement ou sensiblement parallèlement à une surface d'appui d'os (34, 36),
et/ou
b) la au moins une articulation solide (142) relie entre eux en forme de spirale le premier et le second élément d'appui d'os (30, 32).

6. Implant d'articulation selon l'une des revendications précédentes, **caractérisé en ce que** le premier élément d'appui d'os (30) présente au moins deux premiers éléments de support (64) pour des extrémités libres d'une première articulation solide (68, 70), **en ce que** le second élément d'appui d'os (32) présente au moins deux seconds éléments de support (66) pour des extrémités libres d'une seconde articulation solide (68, 70) et **en ce que** la première et la seconde articulation solide (68, 70) se croisent ou se pénètrent ou sont reliées l'une à l'autre dans un domaine de croisement (76).

7. Implant d'articulation selon la revendication 6, **caractérisé en ce que**
a) les premiers et/ou seconds éléments de support (64, 66) sont formés sous forme de protubérances faisant saillie du premier ou second élément d'appui d'os (30, 32) et/ou
b) les premiers et seconds éléments de support (64, 66) forment chacun une partie d'une paroi latérale (38, 40, 42) périphérique au moins par segments du premier élément d'appui d'os (30) ou du second élément d'appui d'os (32) et **en ce que** les parois latérales (38, 40, 42) du premier et second élément d'appui d'os (30, 32) définissent ensemble une douille divisée par une fente périphérique (44),
où en particulier une distance (50, 52) de la fente (44) d'avec le premier élément d'appui d'os (30) et/ou d'avec le second élément d'appui d'os (32) varie en fonction d'un angle périphérique
et/ou
des surfaces de bordure (92, 94) des parois latérales (40, 42) limitant la fente (44) définissent des surfaces de butée (96, 98) pour limiter un mouvement relatif des premier et second éléments d'appui d'os (30, 32),
et/ou
c) chaque élément de support (64, 66) est relié à au moins deux autres éléments de support (64, 66) par des articulations solides (68, 70)
et/ou
d) le premier et/ou le second élément d'appui d'os (30, 32) comprend au moins un évidement de guidage (88, 90) pour un élément de support (64, 66) correspondant de l'autre élément d'appui d'os (30, 32),
où en particulier
d1) le au moins un évidement de guidage (88, 90) est formé sous forme d'une rainure (128)
et/ou
d2) le au moins un évidement de guidage (88, 90) est courbé de manière concave en étant tourné en direction de l'élément de support (64, 66) de l'autre élément d'appui d'os (30, 32), où en particulier le au moins un élément de ressort (116, 118) est formé sous forme d'un ressort à boudin (120, 122) ou sous forme d'un ressort de torsion
et/ou
le au moins un élément de ressort (116, 118, 136) comprend deux ou plus de deux membres de ressort (124, 134), en particulier entrelacés.

8. Implant d'articulation selon l'une des revendications précédentes, **caractérisé en ce que** la au moins une articulation solide (138) comprend une barre de liaison (132).

9. Implant d'articulation selon la revendication 8, **caractérisé en ce que**
a) la barre de liaison (132) est formée en faisant saillie en biais, en particulier perpendiculairement, du premier et/ou second élément d'appui d'os (30, 32)
et/ou
b) la barre de liaison (132) va en se rétrécissant depuis le premier et/ou second élément d'appui d'os (30, 32).

10. Implant d'articulation selon l'une des revendications précédentes, **caractérisé en ce que**
a) le premier et/ou le second élément d'appui d'os (30, 32) est formé sous forme d'un disque plat
et/ou
b) les au moins deux articulations solides (68, 70), en particulier qui se croisent, présentent une section transversale identique
et/ou
c) une section transversale de la au moins une articulation solide (68, 70, 138, 142) est en forme de croix, en forme d'étoile, polygonale, ronde ou ovale.

11. Implant d'articulation selon l'une des revendications précédentes, **caractérisé en ce qu'**un matériau de remplissage est introduit entre le premier et le second élément d'appui d'os (30, 32),
où en particulier le matériau de remplissage
a) est élastique
et/ou
b) est une matière synthétique, en particulier un silicone.

12. Implant d'articulation selon l'une des revendications précédentes, **caractérisé en ce que** la au moins une articulation solide (68, 70, 138, 142) est entourée par une enveloppe (146) ou par une capsule, où en particulier l'enveloppe (146) ou la capsule et/ou l'enveloppe d'articulation (150) ou la capsule d'articulation sont remplies d'un fluide, en particulier d'un liquide compatible avec le corps.

13. Implant d'articulation selon l'une des revendications précédentes, **caractérisé en ce que** l'implant d'articulation comprend une enveloppe d'articulation externe (150) ou une capsule d'articulation externe,
où en particulier le premier élément d'appui d'os (30) présente au moins deux premiers éléments de support (64) pour des extrémités libres d'une première articulation solide (68, 70), **en ce que** le second élément d'appui d'os (32) présente au moins deux seconds éléments de support (66) pour des extrémités libres d'une seconde articulation solide (68, 70) et **en ce que** la première et la seconde articulation solide (68, 70) se croisent ou se pénètrent ou sont reliées l'une à l'autre dans un domaine de croisement (76), **en ce que** les premiers et seconds éléments de support (64, 66) forment chacun une partie d'une paroi latérale (38, 40, 42) périphérique au moins par segments du premier élément d'appui d'os (30) ou du second élément d'appui d'os (32), **en ce que** les parois latérales (38, 40, 42) du premier et du second élément d'appui d'os (30, 32) définissent ensemble une douille divisée par une fente périphérique (44) et **en ce que** l'enveloppe d'articulation (150) ou capsule d'articulation ferme la fente périphérique (44).

14. Implant d'articulation selon l'une des revendications précédentes, **caractérisé en ce que** l'implant d'articulation (12) est fabriqué en un métal ou matière synthétique implantable,
où en particulier le métal est ou contient un acier pour implant, en particulier un acier au cobalt et au chrome, ou du titane et **en ce que** la matière synthétique est ou contient une polyétheréthercétone (PEEK), un polyéthylène (PE) et/ou un silicone.

15. Implant d'articulation selon l'une des revendications précédentes, **caractérisé en ce que** l'implant d'articulation (12)
a) est fabriqué par moulage par injection et/ou impression 3D et/ou
b) est formé sous forme d'une articulation de la cheville artificielle (14), d'un implant intervertébral (20), d'une articulation de la hanche, d'une articulation du genou, d'une articulation de l'épaule, d'une articulation d'un doigt ou d'un orteil.
